# EUROPEAN PATENT SPECIFICATION

(11) **EP 0 535 150 B1**
(45) Date of publication and mention of the grant of the patent: **01.02.1995**
(21) Application number: 91913167.2
(22) Date of filing: 24.06.1991
(51) Int. Cl.: C07K 14/52, C07K 16/24, A61K 38/19

(54) **HUMAN MACROPHAGE INFLAMMATORY PROTEIN 2-BETA**
MENSCHLICHES MACROPHAGEENTZÜNDUNGSPROTEIN-2-BETA
PROTEINE INFLAMMATOIRE 2-BETA PRODUITE PAR LES MACROPHAGES HUMAINS

(30) Priority: 22.06.1990 US 541898; 19.06.1991 US 715195
(43) Date of publication of application: 07.04.1993
(73) Proprietor: CHIRON CORPORATION, Emeryville, California 94608 (US)
(72) Inventor: TEKAMP-OLSON, Patricia, San Anselmo, CA 94960 (US); GALLEGOS, Carol, Ann, Albany, CA 94706 (US)
(74) Representative: Hallybone, Huw George
(86) International application number: US9104478
(87) International publication number: WO9200326

(56) References cited:
- WO-A-90/02762
- Dialog Information Services, File 154, Medline 85-91, Dialog accession no. 7447792, Tekamp-Olson p et al.: "Cloning and characterization of cDNAs for murine macrophage inflammatory protein 2 and its human homologues", & J. Exp Med Sept 1 1990, 172 (3) p911-9.
- Proc. Natl. Accad. Sci. , vol. 86, January 1989, Stephen D. Volpe et al: "Identification and characterization of macrophage inflammatory protein 2 ", see page 612 - page 616.
- Dialog Information Services, File 55, BIOSIS 85-91, Dialog accession no. 7364087, Broxmeyer H E et al: "Myelopoietic enhanching effects of murine macrophage inflammatory proteins 1 and 2 on colony formation in-vitro by murine and human bone marrow granulocyte-macrophage progenitor cells", & J Exp Med 170 (5). 1989. 1583-1594.
- Nucleic Acids Research, vol., 18, no. 21, September 1990, Nancy E. Baker et al.: "Nucleotide sequence of the human melanoma growth stimulatory activity (MGSA) gene", see page 6453.
- Proc Natl. Acad. Sci. , vol., 87, October 1990, Stephen Haskill et al.: "Identification of three related human GRO genes encoding cytokine functions", see page 7732 - page 7736.

## Description

### BACKGROUND OF THE INVENTION

Macrophage Inflammatory Proteins (MIPs) represent a class of proteins that are produced by certain cells (for example, macrophages or lymphocytes) in response to invasive stimuli such as gram negative bacterial lipopolysaccharide. Thus, they may be important participants in the response of the cell (host organism) to such stimuli. As such, these molecules may have therapeutic potential in the treatment of infections, cancer, myelopoietic dysfunction and auto-immune diseases. Two distinct forms of MIP have been found in cultures of macrophage tumor cells from the mouse: MIP-1 and MIP-2.

### Murine MIP-1

Murine (m)MIP-1 is a major secreted protein from lipopolysaccharide (LPS) - stimulated RAW 264.7 cells, a murine macrophage tumor cell line. It has been purified and found to consist of two related proteins, mMIP-1α and mMIP-1β (Wolpe, et al., 1987, J. Exp. Med., 167:570; Sherry, et al., 1988, J. Exp. Med., 168:2251).

The cDNA's for both mMIP-1α and mMIP-1β have been cloned and sequenced (Davatelis, et al., 1988, J. Exp. Med., 167:1939; Sherry, et al., op. cit.) The cloning and sequencing of cDNAs corresponding to mMIP-1α and mMIP-1β have been reported as well by Brown, et al. (1989, J. Immunol., 142:679); Kwon and Weissman (1989, Proc. Natl. Acad. Sci. USA, 86:1963) and by Brown, et al., op. cit., respectively. Both groups isolated homologs of mMIP-1α and/or mMIP-1β from cDNA libraries prepared from RNA of murine helper T-cells that had been activated by treatment with conconavalin A. These results suggest that MIP-1α and MIP-1β may play a role in T-cell activation.

### Human MIP-1 Homologs

Several groups have cloned may be the human homologs of mMIP-1α and mMIP-1β. In all cases, cDNAs were isolated from libraries based on RNA from activated human T-cells. Thus Obaru et al., (1986, J. Biochem., 99:885) and Zipfel, et al. (1989, J. Immunol., 142:1582) have both reported cloning of a cDNA that predicts a protein with high homology to mMIP-1α (76%). Similarly, Brown, et al., op. cit., Zipfel, et al., op. cit., Lipes, et al. (1988, Proc. Natl. Acad. Sci. USA, 85:9704) and Miller, et al. (1989, J. Immunol., 143:2907) have reported the cloning and sequencing of human cDNAs, which predict a protein with high homology to mMIP-1β (75%). MIP-1α and MIP-1β belong to a newly described family of related proteins which have immunomodulatory activities (see Sherry, et al., op. cit. for a review).

### Murine MIP-2

Murine MIP-2 (mMIP-2) is an inducible protein that has also been purified from the conditioned medium of LPS-stimulated RAW 264.7 cells (Wolpe, et al., 1989, Proc. Natl. Acad. Sci. USA, 86:3121). The cDNA encoding murine MIP-2 has been cloned and sequenced.

mMIP-2 also is a member of a homologous multigene family. Members of this family include gro/MGSA, platelet factor 4, platelet basic protein, the precursor of connective tissue activating peptide (CTAP-III) and B-thromboglobulin, a gamma interferon inducible protein, IP-10, and interleukin 8 (IL-8), also known as 3-10C, MDNCF, NAP-1 and NAF. Members of this family that have highest homology in protein sequence (generally predicted from cloned cDNA) include MGSA and KC. MGSA (Richmond, et al., 1988, EMBO J., 7:2025) is an autocrine growth factor with mitogenic activity secreted by human melanoma cells and is the product of the human gro gene (Anisowicz, et al., 1987, Proc. Natl. Acad. Sci. USA, 84:7188). MGSA has 57.9% identity in amino acid sequence to murine MIP-2; the predicted protein sequence of the putative hamster homolog of MGSA (ibid.) has 68.3% identity to mMIP-2. The murine KC gene product (Oquendo, et al., 1989, J. Biol. Chem., 264:4233) is induced by platelet-derived growth factor (PDGF) and is thought to be the murine homolog of the human MGSA/gro gene (63.0% amino acid identity to mMIP-2).

### Recombinant Expression of MIP

There is no prior art on the expression of recombinant MIP-2, or human MIP-2α and MIP-2β although members of the MIP-2 gene family as well as members of the related MIP-1 gene family have been expressed. The pertinence of these results to the expression of murine or human MIP-2 is questionable, given the idiosyncratic nature of expression in heterologous systems. The literature will be summarized nonetheless.

mMIP-1α and mMIP-1β have been independently expressed in COS cells (Graham, et al., 1990, Nature, 344:442). LD78 cDNA (Obaru, et al., op. cit.) which encodes a protein that is likely to be the human homolog of murine MIP-1α has been expressed in E. coli as a carboxyl terminal fusion to human interleukin-2, as well as in COS cells (Yamamura, et al., 1989, J. Clin. Invest., 84:1707). Human I-309, a cDNA that encodes a protein with homology to members of the MIP-1 family of proteins, has been expressed in COS-1 cells in order to confirm that it encodes a secreted protein (Miller, et al., op. cit.). Lipes, et al., (op. cit.) described baculovirus expression of Act-2 cDNA, the human homolog of murine MIP-1β, to show that the protein encoded was secreted and to identify the mature N-terminal sequence. JE, a murine cDNA that encodes a protein with homology to MIP-1α and MIP-1β, has been expressed in COS-1 cells to confirm that it encodes a polypeptide core of about 12 kDa (Rollins, et al., 1988, Proc. Natl. Acad. Sci. USA, 85:3738).

KC, a murine cDNA that encodes a protein with homology to mMIP-2, has been expressed in COS-1 cells to show that it encodes a secreted protein (Oquendo, et al., op. cit.) Connective tissue activating peptide-III (CTAP, Mullenbach, et al., 1986, J. Biol Chem., 261:719) and IP-10, (Luster and Ravetch (1987, J Exp. Med., 166:1084) both members of the MIP-2 gene family, have been expressed in yeast as an α-factor fusion and in E. coli, respectively. Maione, et al. (1990, Science, 247:77) expressed human platelet factor 4, (MIP-2 family) in E. coli as a peptide fused to a 35 amino acid peptide fragment of E. coli β-glucuronidase. The insoluble fusion protein must be cleaved with cyanogen bromide in order to generate bioactive material. Lindley, et al., (1988, Proc. Natl. Acad. Sci. USA, 85:9199) have expressed NAF (IL-8), a member of the MIP-2 family, in E. coli. After purification and renaturation, this recombinant protein was found to have the same bioactivity as that identified for the native molecule. Furuta, et al. (1989, J. Biochem., 106:436) have also expressed IL-8 (MDNCF) in E. coli. Finally, Gimbrone, et al. (1989 Science, 246:1601) have expressed endothelial IL-8 in human 293 cells and shown that the recombinant and natural material have the same bioactivity.

### Bioactivity of MIP

Studies on the bioactivities of MIP-1 and -2 are in progress and have utilized native murine MIP-1 or MIP-2 and very recently recombinant murine (rm) MIP-1α, rmMIP-1β and rmMIP-2. Among the bioactivities defined for both recombinant and native mMIP-1 and mMIP-2 is colony-stimulating-factor promoting activity as well as roles in inflammation.

Murine MIP-2 has been shown to elicit a localized inflammatory response when injected subcutaneously into the footpads of C3H/HeJ mice, to have potent chemotactic activity for human polymorphonuclear leukocytes (PMN), and to induce PMN degranulation of lysozyme but not of β-glucuronidase (Wolpe, et al., 1989). In addition, mMIP-2 has been shown to have myelopoietic enhancing activities for CFU-GM (Broxmeyer, et al., 1989, J. Exp. Med., 170:1583). Given the various biological activities of these factors, it is desirable to isolate their human homologs. Due to the necessity for quantities of purified factors to pursue definition of bioactivities and the expense of isolating these factors from cell culture fluids it is desirable to produce these materials by employing recombinant DNA technology.

### SUMMARY OF THE INVENTION

An attempt to Identify the human counterpart of mMIP-2 unexpectedly resulted in not one, but two candidate sequences. These two sequences were designated hu-MIP-2α and hu-MIP-2β, respectively.

It is an object of this invention to provide a human MIP-2β (hu-MIP-2β) polypeptide substantially free from other human proteins and to provide a nucleic acid coding sequence for hu-MIP-2β.

It is yet another object of this invention to provide antibodies reactive with hu-MIP-2β.

It is still another object of this invention to provide diagnostic methods for detection of hu-MIP-2β or of nucleic acid coding sequences for hu-MIP-2β.

In accordance with these and other objects, which will become apparent from the description herein, one aspect of this invention contemplates a protein composition comprising hu-MIP-2β polypeptides wherein the composition is substantially free of human tissue and preferably is substantially free of other human proteins.

In another aspect, the invention contemplates a DNA molecule comprising a heterologous region that encodes hu-MIP-2β polypeptide. In yet another aspect, the invention contemplates a recombinant production system comprising cells transformed with a DNA molecule comprising a heterologous region that encodes hu-MIP-2β polypeptide.

The invention also contemplates antibodies which are reactive with hu-MIP-2β polypeptides. Still further aspects of the invention relate to immunoassay methods for determination of hu-MIP-2β in tissues or fluids and nucleic acid probe methods for detecting polynucleotide sequences coding for hu-MIP-2β in biological samples.

Another aspect of the invention contemplates polypeptides and small organic molecules that interact with the hu-MIP-2β receptor(s) and possess agonistic or antagonistic activities.

Yet another aspect of the invention contemplates hu-MIP-2β polypeptides that exhibit agonist and antagonist activities to any of the members of the homologous multigene family.

Further, the invention contemplates a method of inducing wound healing, a method of modulating myelopoeisis, and a method of inducing adjuvant activity, by administering an effective amount of hu-MIP-2β polypeptides.

The invention also contemplates a method of treating malignancy, autoimmune and inflammatory diseases, such as, pathological infiltration of neutrophils including psoriasis, rheumatoid arthritis, and diseases of the lungs, by administering an effective amount of hu-MIP-2β antagonists or hu-MIP-2β polypeptides with antagonistic activity to the members the homologous multigene family.

By using the DNA sequences and the recombinant system provided by this invention, it is possible to produce large quantities of one of the two human homologs of mMIP-2 substantially free of other human protein, because only one human protein is expressed in the recombinant system. Compositions such as this, containing only one MIP-2 homolog, would be difficult to obtain by purification from natural sources.

### BRIEF DESCRIPTION OF THE DRAWINGS

Figure 1. Nucleotide sequence and predicted protein sequence of murine MIP-2.

Figure 2. Nucleotide sequence and predicted protein sequence of hu-MIP-2α.

Figure 3. Nucleotide sequence and predicted protein sequence of hu-MIP-2β.

Figure 4. Nucleotide sequence homology of MIP-2 homologs. Percentages of nucleotide sequence identity between mMIP-2, hu-MIP-2α, hu-MIP-2β, hu-gro/MGSA and murine KC in the cDNA (A), coding region (B) and 3' untranslated region (C).

Figure 5. Amino acid homology and alignment of MIP-2 homologs and human IL-8. (A) Percentages of identity between the predicted amino acid sequences of MIP-2 homologs as well as human IL-8. (B) The aligned amino acid sequences.

Figure 6. Southern analysis of genomic DNA with murine and human MIP-2 cDNA digested with BamH1, 'B'; EcoR1, 'E' or EcoRV, 'R'. (A) Murine DNA hybridized with mMIP-2 cDNA. A blot of restricted human DNA was hybridized first with labelled hu-MIP-2β cDNA (C) then the filter was stripped and rehybridized with labelled hu-MIP-2α cDNA (B).

### DETAILED DESCRIPTION

The practice of the present invention employs, unless otherwise indicated, conventional molecular biology, microbiology, and recombinant DNA techniques within the skill of the art. Such techniques are explained fully in the literature. See, e.g., Maniatis, Fritsch & Sambrook, "Molecular Cloning: A Laboratory Manual" (1982); "DNA Cloning: A Practical Approach," Volumes I and II (D.N. Glover, ed., 1985); "Oligonucleotide Synthesis" (M.J. Gait, ed., 1984); "Nucleic Acid Hybridization" (B.D. Names & S.J. Higgins, eds., 1985); "Transcription and Translation" (B.D. Names & S.J. Higgins, eds., 1984); "Animal Cell Culture" (R.I. Freshney, ed., 1986); "Immobilized Cells and Enzymes" (IRL Press, 1986); B. Perbal, "A Practical Guide to Molecular Cloning" (1984).

### Definitions

In describing the present invention, the following terminology is used in accordance with the definitions set out below.

A "replicon" is any genetic element (e.g., plasmid, chromosome, virus) that functions as an autonomous unit of DNA replication in vivo; i.e., capable of replication under its own control.

A "vector" is a replicon, such as plasmid, phage or cosmid, to which another DNA segment may be attached so as to bring about the replication of the attached segment.

A "double-stranded DNA molecule" refers to the polymeric form of deoxyribonucleotides (adenine, guanine, thymine, or cytosine) in its normal, double-stranded helix. This term refers only to the primary and secondary structure of the molecule, and does not limit it to any particular tertiary forms. Thus, this term includes double--stranded DNA found, inter alia, in linear DNA molecules (e.g., restriction fragments), viruses, plasmids, and chromosomes. In discussing the structure of particular double-stranded DNA molecules, sequences may be described herein according to the normal convention of giving only the sequence In the 5' to 3' direction along the nontranscribed stand of DNA (i.e., the strand having a sequence homologous to the mRNA).

A DNA "coding sequence" is a DNA sequence which is transcribed and translated into a polypeptide in vivo when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, procaryotic sequences, cDNA from eucaryotic mRNA, genomic DNA sequences from eucaryotic (e.g., mammalian) DNA, and even synthetic DNA sequences. A polyadenylation signal and transcription termination sequence will usually be located 3' to the coding sequence.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the translation start codon of a coding sequence and extends upstream (5' direction) to include the minimum number of bases or elements necessary to initiate transcription at levels detectable above background. Within the promoter sequence will be found a transcription initiation site (conveniently defined by mapping with nuclease S1), as well as protein binding domains (consensus sequences) responsible for the binding of RNA polymerase. Eucaryotic promoters will often, but not always, contain "TATA" boxes and "CAT" boxes. Procaryotic promoters contain Shine-Delgarno sequences in addition to the -10 and -35 consensus sequences.

A coding sequence is "under the control" of the promoter sequence in a cell when RNA polymerase which binds the promoter sequence transcribes the coding sequence into mRNA which is then in turn translated into the protein encoded by the coding sequence.

A cell has been "transformed" by exogenous DNA when such exogenous DNA has been introduced inside the cell wall. Exogenous DNA may or may not be integrated (covalently linked) to chromosomal DNA making up the genome of the cell. In procaryotes and yeast, for example, the exogenous DNA may be maintained on an episomal element such as a plasmid. With respect to eucaryotic cells, a stably transformed cell is one in which the exogenous DNA has become integrated into a chromosome so that it is inherited by daughter cells through chromosome replication. This stability is demonstrated by the ability of the eucaryotic cell to establish cell lines or clones comprised of a population of daughter cells containing the exogenous DNA. A "clone" is a population of cells derived from a single cell or common ancestor by mitosis. A "cell line" is a clone of a primary cell that is capable of stable growth in vitro for many generations.

Two DNA sequences are "substantially homologous" when at least about 85% (preferably at least about 90%, and most preferably at least about 95%) of the nucleotides match over the defined length of the DNA sequences. Sequences that are substantially homologous can be identified in a Southern hybridization experiment under, for example, stringent conditions as defined for that particular system. Defining appropriate hybridization conditions is within the skill of the art. See e.g., Maniatis et al., supra; DNA Cloning, vols. 1 and II supra; Nucleic Acid Hybridization, supra.

A "heterologous" region or domain of a DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. Another example of a heterologous region is a construct where the coding sequence itself is not found in nature (e.g., a cDNA where the genomic coding sequence contains introns, or synthetic sequences having codons different than the native gene). Allelic variations or naturally occurring mutational events do not give rise to a heterologous region of DNA as defined herein.

The terms "analyte polynucleotide" and "analyte strand" refer to a single- or double-stranded nucleic acid molecule which is suspected of containing a target sequence, and which may be present in a biological sample.

As used herein, the term "probe" refers to a structure comprised of a polynucleotide which forms a hybrid structure with a target sequence, due to complementarity of at least one sequence in the probe with a sequence in the target region. The polynucleotide regions of probes may be composed of DNA, and/or RNA, and/or synthetic nucleotide analogs.

As used herein, the term "target region" refers to a region of the nucleic acid which is to be amplified and/or detected. The term "target sequence" refers to a sequence with which a probe or primer will form a stable hybrid under desired conditions.

The term "targeting polynucleotide sequence" as used herein, refers to a polynucleotide sequence which is comprised of nucleotides which are complementary to a target nucleotide sequence; the sequence is of sufficient length and complementarity with the target sequence to form a duplex which has sufficient stability for the purpose intended.

The term "binding partner" as used herein refers to a molecule capable of binding a ligand molecule with high specificity, as for example an antigen and an antibody specific therefor. In general, the specific binding partners must bind with sufficient affinity to immobilize the analyte (or the copy/complementary strand duplex, in the case of capture probes) under the isolation conditions. Specific binding partners are known in the art, and include, for example, biotin and avidin or streptavidin, IgG and protein A, the numerous known receptor-ligand couples, and complementary polynucleotide strands. In the case of complementary polynucleotide binding partners, the partners are normally at least about 15 bases in length, and may be least 40 bases in length; in addition, they generally have a content of Gs and Cs of at least about 40% and as much as about 60%. The polynucleotides may be composed of DNA, RNA, or synthetic nucleotide analogs.

The term "coupled" as used herein refers to attachment by covalent bonds or by strong non-covalent interactions (e.g., hydrophobic interactions, hydrogen bonds, etc.). Covalent bonds may be, for example, ester, ether, phosphoester, amide, peptide, imide, carbon-sulfur bonds, carbon-phosphorus bonds, and the like.

The term "support" refers to any solid or semisolid surface to which a desired binding partner may be anchored. Suitable supports include glass, plastic, metal, polymer gels, and the like, and may take the form of beads, wells, dipsticks, membranes, and the like.

The term "label" as used herein refers to any atom or moiety which can be used to provide a detectable (preferably quantifiable signal, and which can be attached to polynucleotide or polypeptide.

As used herein, the term "label probe" refers to a polynucleotide which is comprised of targeting polynucleotide sequence that is complementary to a target sequence to be detected in the analyte polynucleotide. This complementary region is of sufficient length and complementarity to the target sequence to afford a duplex comprised of the "label probe" and the "target sequence" to be detected by the label. It is coupled to a label either directly, or indirectly via a set of ligand molecules with high specificity for each other. Sets of ligand molecules with high specificity are described supra (see "binding partners").

As used herein, a "biological sample" refers to a sample of tissue or fluid isolated from a individual, including but not limited to, for example, plasma, serum, spinal fluid, lymph fluid, the external sections of the skin, respiratory, intestinal, and genitourinary tracts, tears, saliva, milk, blood cells, tumors, organs, and also samples of in vivo cell culture constituents (including but not limited to conditioned medium resulting from the growth of cells in cell culture medium, putatively virally infected cells, recombinant cells, and cell components).

"Human tissue" is an aggregate of human cells which may constitute a solid mass. This term also encompasses a suspension of human cells, such as blood cells, or a human cell line.

A composition comprising a selected component A is "substantially free" of another component B when component A makes up at least about 75% by weight of the combined weight of components A and B. Preferably, selected component A comprises at least about 90% by weight of the combined weight, most preferably at least about 99% by weight of the combined weight. In the case of a composition comprising a selected biologically active protein, which is substantially free of contaminating proteins, it is sometimes preferred that the composition having the activity of the protein of interest contain species with only a single molecular weight (i.e., a "homogeneous" composition).

Two amino acid sequences are "substantially homologous" when at least about 90% of the amino acids match over the defined length of the amino acid sequences, preferably a match of at least about 92%, more preferably a match of at least about 95%.

### Human Homologs of Murine MIP-2

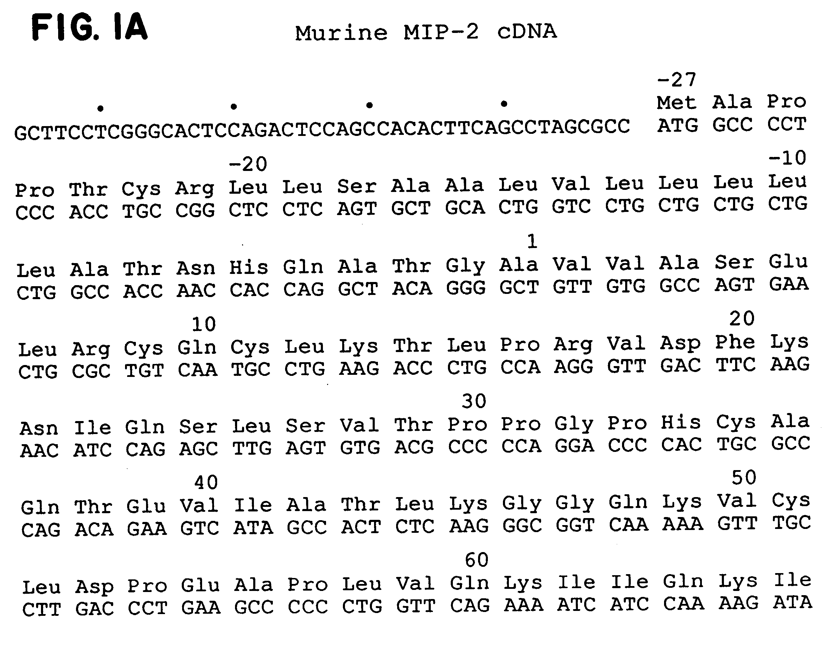

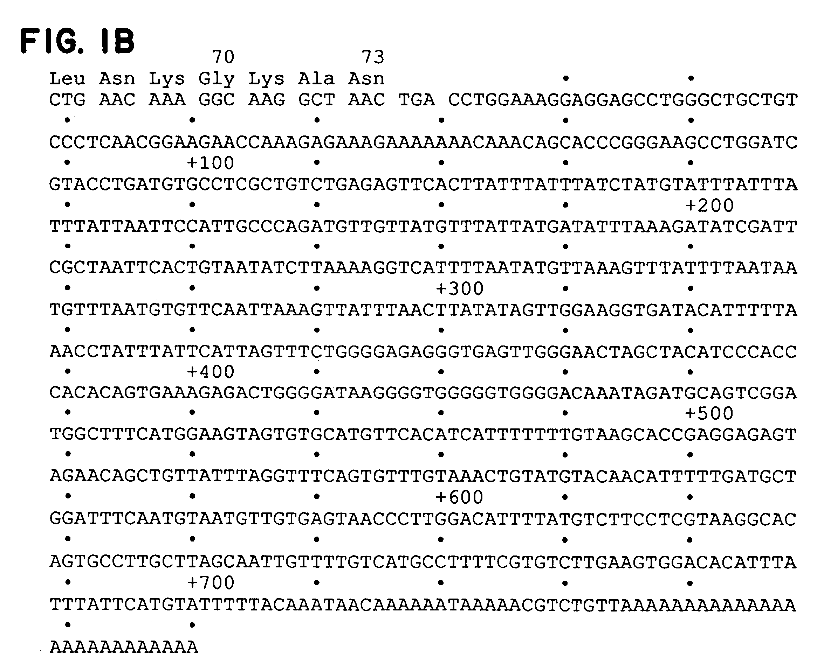

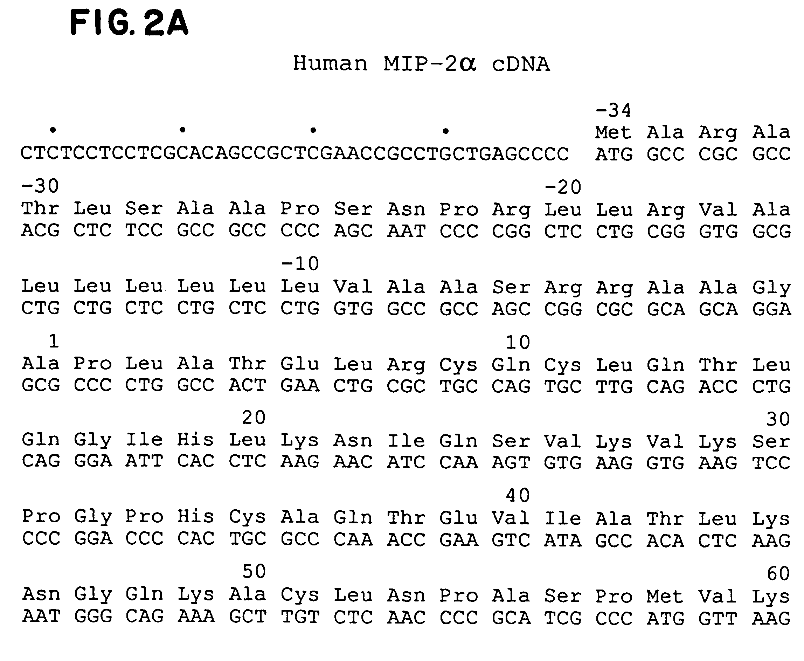

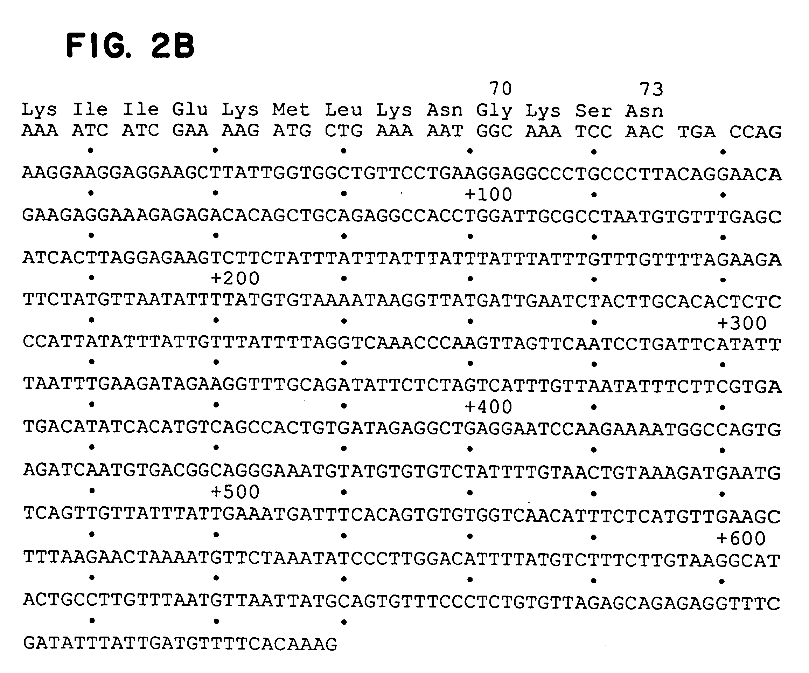

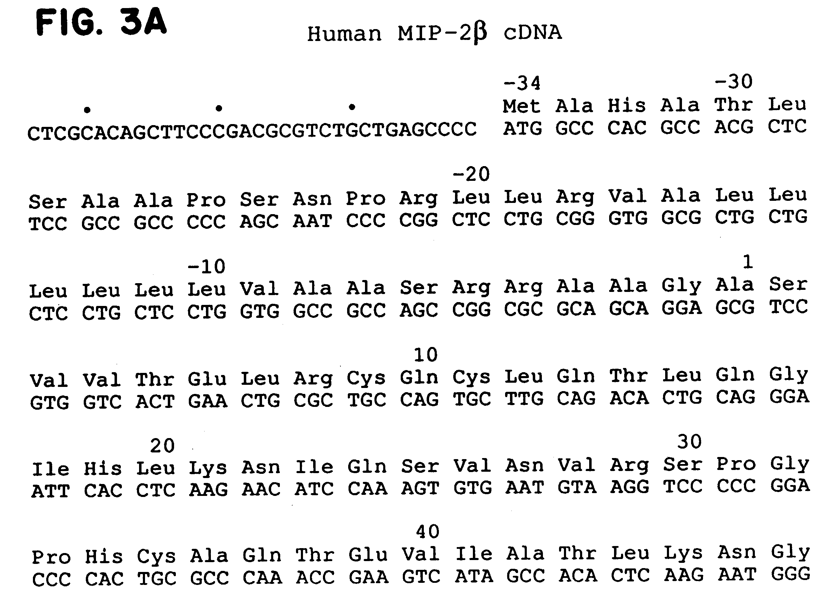

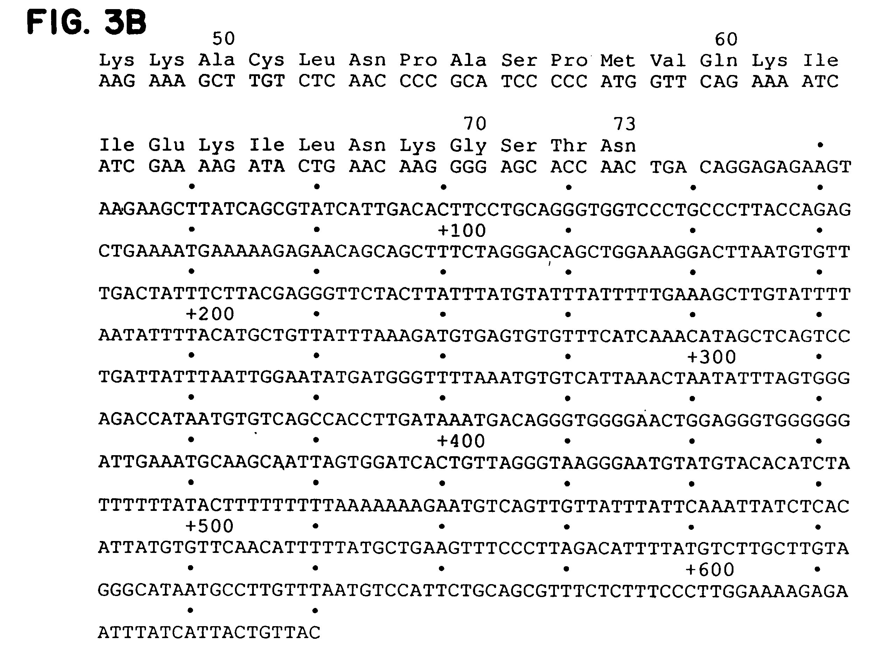

The cDNA for murine MIP-2 has been cloned using a degenerate oligonucleotide probe pool corresponding to a portion of the N-terminal amino acid sequence determined on the purified protein. When nucleotide probes representing the coding region of murine MIP-2 cDNA were used to probe a cDNA library from human macrophage cell line stimulated with LPS (see Example 2) two different candidate cDNA sequences were identified. The proteins encoded by these two cDNA sequences have been designated hu-MIP-2α and hu-MIP-2β, respectively. Examples of the nucleic acid and amino acid sequences of these three proteins are shown in the Figures: Figure 1 = murine MIP-2, Figure 2 = human MIP-2α, Figure 3 = human MIP-2β.

"Human macrophage inflammatory protein 2β polypeptides" (hu-MIP-2β polypeptides) encompass hu-MIP-2β and hu-MIP-2β analogs.

"Hu-MIP-2β" is human macrophage inflammatory protein 2β, a naturally occurring, mature human protein secreted, inter alia, by LPS-stimulated macrophages, and further encompasses all precursors and allelic variations of hu-MIP-2β, and as well as including forms of heterogeneous molecular weight that may result from inconsistent processing in vivo. An example of the hu-MIP-2β sequence is shown in Figure 2.

"Hu-MIP-2β analogs" are a class of peptides which includes:
1) "Hu-MIP-2β muteins," which are polypeptides substantially homolgous to hu-MIP-2β. Preferably the amino acid sequence of the "mutein" differs from that of hu-MIP-2β by 8 or fewer amino acid residues, more preferably, 7 or fewer residues, even more preferably about 5 or fewer residues and most preferably about 2 or fewer residues. It is sometimes preferred that any differences in the amino acid sequences of the two proteins involve only conservative amino acid substitutions. Conservative amino acid substitutions occur when an amino acid has substantially the same charge as the amino acid for which it is substituted and the substitution has no significant effect on the local conformation of the protein. Alternatively, changes such as the elimination of cysteine which alter the activity or stability of the protein may be preferred.
2) "Truncated hu-MIP-2β peptides," which include fragments of either "hu-MIP-2β" or "hu-MIP-2β muteins" that preferably retain either (i) an amino acid sequence unique to hu-MIP-2β, (ii) an epitope unique to hu-MIP-2β or (iii) MIP-2 activity,
3) "Hu-MIP-2β fusion proteins," which include heterologous polypeptides, are made up of one of the above polypeptides (hu-MIP-2β, hu-MIP-2β muteins or truncated hu-MIP-2β peptides) fused to any heterologous amino acid sequence. Preferably such heterologous sequences are fused to the N-terminal end of the hu-MIP sequence and comprise a leader sequence to direct secretion.

"Unique" hu-MIP-2β sequences, either amino acid sequences or nucleic acid sequences which encode them, are sequences which are identical to a sequence of a hu-MIP-2β polypeptide, but which differ in at least one amino acid or nucleotide residue from the sequences of hMGSA, hu-MIP-2α, mMIP-2 and murine KC, and preferably, are not found elsewhere in the human genome. Similarly, an epitope is "unique" to hu-MIP-2β polypeptides if it is found on hu-MIP-2β polypeptides but not found on any members of the homologous gene family.

### Features of the Predicted Amino Acid Sequence

The open reading frames of mMIP-2, hu-MIP-2α and hu-MIP-2β encode polypeptides of 100, 107 and 107 amino acids, respectively. The initial approximately 30 amino acids of each of the three polypeptides have characteristic features of a signal sequence (Perlman, et al., 1983, J. Mol. Biol., 167:391; von Heijne, 1984, J. Mol. Biol., 173:243; von Heijne, 1986, Nucleic Acids Res., 14:4683). The N-terminal amino acid sequence determined for secreted mMIP-2 purified from the conditioned medium of LPS-stimulated RAW 264.7 cells (Wolpe, et al., 1989) determined the start of the mature mMIP-2 protein in the predicted amino acid sequence. The start of the predicted mature peptide sequence for hu-MIP-2α and hu-MIP-2β aligns with that of mMIP-2 and has a consensus signal peptide cleavage site (Perlman, et al. 1983; von Heijne, 1984, 1986). The predicted length of the mature peptide sequence for all three proteins is 73 amino acids. Murine MIP-2 is a basic protein, and the human MIP-2 polypeptides are basic as well, based on predicted isoelectric points of 9.9 and 9.7 for hu-MIP-2α and hu-MIP-2β, respectively. The native or recombinant proteins may be O-glycosylated. None of the three predicted polypeptides has a consensus signal for N-linked glycosylation.

### Features of Murine and Human MIP-2 cDNAs

The nucleotide sequences in cDNA's for mMIP-2, hu-MIP-2α and hu-MIP-2β each encode a single open reading frame. The nucleotide sequence environment of the initiating ATG codon of mMIP-2 conforms to the consensus sequence shared by many mRNAs of higher eucaryotes (Kozark, 1986, Cell, 44:283; Kozark, 1987, Nucleic Acid Res., 15:8125); those of human MIP-2α and MIP-2β lack the highly conserved purine at position -3 but possess many features of the consensus sequence including C residues at positions -1,-2, and -4 and a G residue at position +4

The 3' untranslated region of mMIP-2 includes the eucaryotic consensus polyadenylation signal AATAAA (Birnstiel, et al., 1985, Cell, 41:349) at position +719-724 followed by a poly-A string beginning at nucleotide +735. No AATAAA polyadenylation signal was found in the 3' untranslated region of clones hu-MIP-2-4a or hu-MIP-2-7d of hu-MIP-2β cDNA or in clones of hu-MIP-2α cDNA. This is most likely due to the fact that these clones have a truncated 3' untranslated region, since no poly-A string was present.

The consensus sequence TTATTTAT found in the 3' untranslated region of many cytokine genes (Caput, et al., 1986, Proc. Natl. Acad. Sci. USA, 83:1670) and implicated in mRNA stability (Shaw, et al., 1986, Cell, 46:639) and efficiency of translation (Kruys, et al., 1987, Proc. Natl. Acad. Sci. USA, 84:6030; Han, et al., 1990, J. Exp. Med., 171:465) is present in multiple copies in all three cDNAs. This sequence is present at four positions, two overlapping, in the 3' untranslated region of mMIP-2 (positions +122, +126, +142, +146); and is present two times (positions +158 and +471) and five times, two overlapping, (positions +148, +152, +156, +160 and +492) in the 3' untranslated regions of human MIP-2β and MIP-2α, respectively.

### Homologs of Murine and Human MIP-2

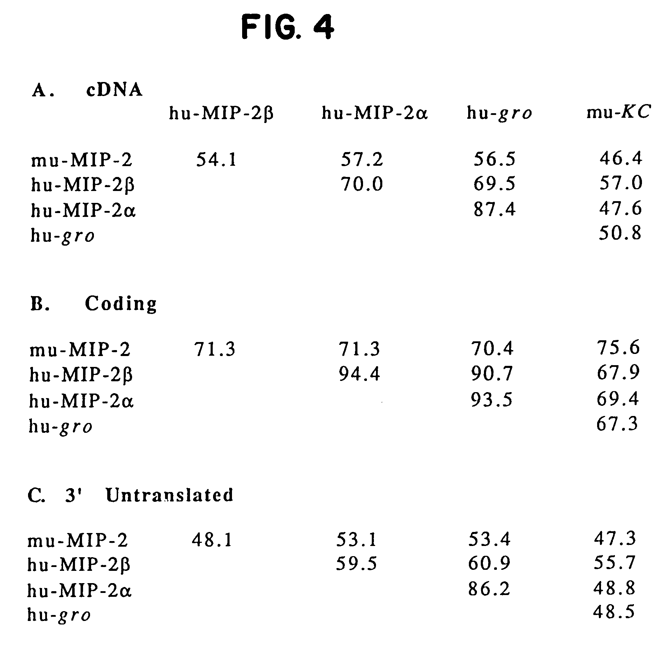

The percentage of nucleotide sequence identity among the three MIP-2 cDNA's, one murine and two human, as well as that of the human gro/MGSA cDNA (Anisowicz, et al., 1987; Richmond, et al., 1988) and the murine KC cDNA (Oquendo, et al., 1989; Cochran, et al., 1983, Cell, 33:939) is displayed in Figure 4A. The human gro/MGSA cDNA encodes a protein with melanoma growth stimulating activity (Richmond, et al., 1986, J. Cell. Physiol., 129:375); murine KC is a platelet-derived growth factor (PDGF)-inducible gene presumed to be the murine homolog of human MGSA. Noteworthy is the high degree of nucleotide homology among the three human cDNAs, particularly between hMGSA and hu-MIP-2α. There is an even more striking degree of nucleotide sequence identity among the three human homologs in the coding region as shown in Figure 4B. The nucleotide sequence identity in the 3' untranslated regions of the human MIP-2 homologs is considerably less than that observed in the coding regions, with the exception of the respective regions of hu-MIP-2α and hMGSA. These two cDNAs show a high percentage of sequence homology throughout the 3' untranslated region as well.

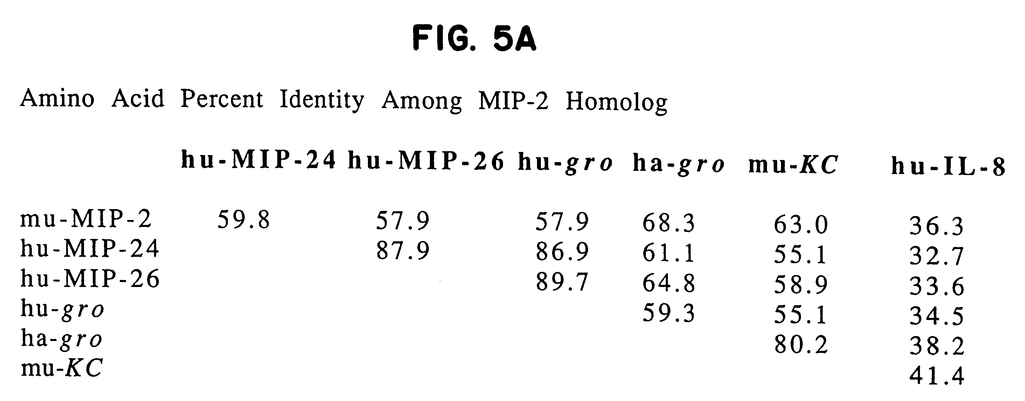

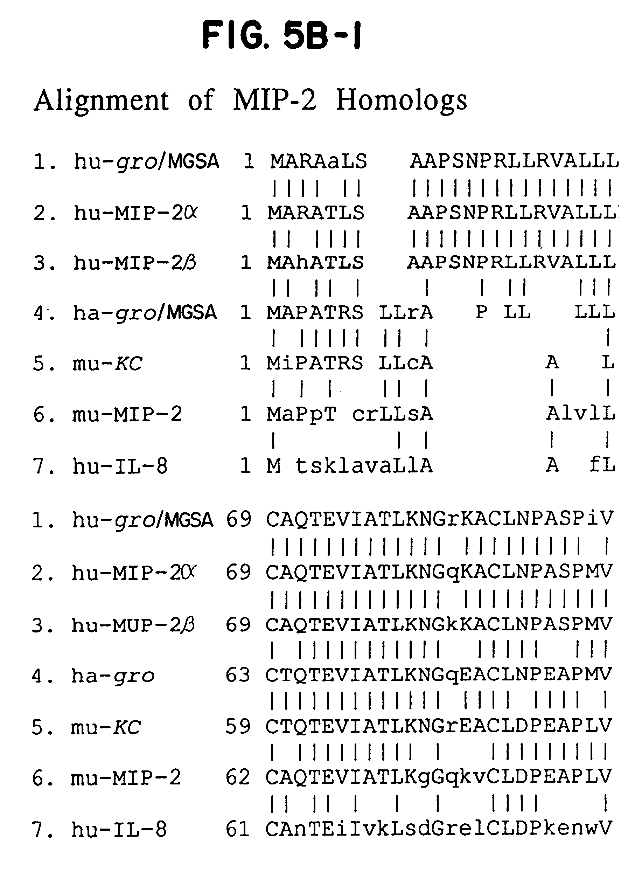

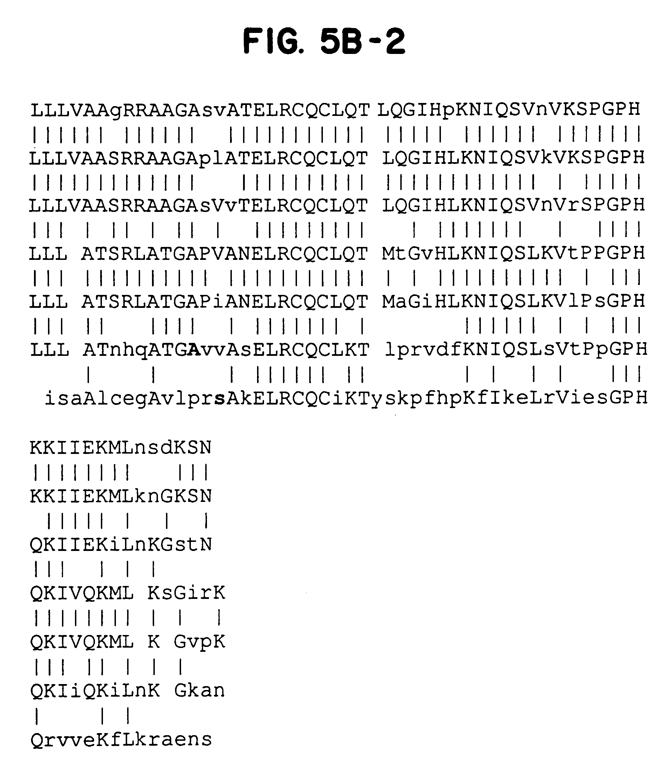

Homology comparisons and alignments of the predicted amino acid sequences of the precursor proteins of MIP-2 homologs including hu-MIP-2α, hu-MIP-2β, human gro/MGSA, the hamster homolog of gro/MGSA (ha-gro), mMIP-2 and mKC are presented in Figure 5(A,B). The three human proteins are highly homologous (87-90%) but amino acid differences occur throughout the predicted sequences, particularly at the carboxy termini of the mature protein sequences. Based on predicted amino acid homologies alone, it is not possible to assign hu-MIP-2α, hu-MIP-2β or human gro/MGSA as the human homolog of mMIP-2 or murine KC.

The percentage of nucleotide sequence identity is greatest between hu-MIP-2α and hMGSA and it extends throughout the entire cDNA. The presence of both hu-MIP-2α and hu-MIP-2β in a U937 cDNA library, prepared using poly-A+ RNA from cells stimulated with both LPS and phorbol myristyl acetate (PMA), prompted us to screen for hMGSA as well. Screening of 5x10⁵ plaques from the library after amplification with oligonucleotides specific for hMGSA (and not hu-MIP-2α/β) gave no positive signals; in contrast 56 hu-MIP-2α positive signals were detected. This suggests that gro/MGSA transcription is not induced in U937 cells stimulated by PMA and LPS, in contrast to transcription of the hu-MIP-2α gene.

### Activities of MIP-2

Murine MIP-2 purified from the conditioned medium of LPS-stimulated RAW 264.7 cells has diverse activities including CSF-dependent myelopoietic enhancing activity for CFU-GM (Broxmeyer, et al., 1989), elicitation of a localized inflammatory response following subcutaneous administration and a potent chemotactic activity for human PMN (Wolpe, et al., 1989). The latter activity is characteristic of human IL-8 (hu-IL-8). Based on this functional equivalence alone it was suggested that mMIP-2 could be the murine homolog of hu-IL-8. However, given that the amino acid homology of mMIP-2 to hu-IL-8 is low, relative to mMIP-2 homology to hu-MIP-2α, hu-MIP-2β or hu-MGSA (Figure 5), mMIP-2 and hu-IL-8 do not appear to be murine/human homologs. Redundancy of function among cytokines is not uncommon; cachectin/TNF-a and interleukin 1 have an overlapping activity profile (Manogue, et al., 1988, in "Cellular and Molecular Aspects of Inflammation," Poste, et al., eds., Plenum, NY, p. 123). MIP-2 and IL-8 may be another example of this functional redundancy.

Based on its cDNA sequence, hu-MIP-2β can be classified as a member of the homologous multigene family which includes murine MIP-2. The members of this gene family possess in vitro biological activities including neutrophil activation, neutrophil chemotaxis, MGSA-like mitogenic activity, fibroblast mitogenic activity, CSF co-factor activity, monocyte chemotaxis, angiogenic activity, and inflammatory activity. Hu-MIP-2β polypeptides may be selected which possess one or more of the biological activities exhibited by any member of the multigene family. Hu-MIP-2β polypeptides may be utilized therapeutically for stimulation of myelopoiesis, as an adjuvant in vaccine formulations, and for wound healing.

Hu-MIP-2β biological activity can be measured by assays described in Wolpe, et al. (1989) or Broxmeyer, et al., (1989) (each incorporated herein by reference). Other assays for hu-MIP-2β activities such as neutrophil activation or chemotaxis can be measured in vitro as described in Waltz et al., (1989), J. Exp. Med., 170:1745, Clark-Lewis et al., (1991), Biochem., 30:3128, and Dernyck et al., (1990), Biochem., 29:10225 (all incorporated herein by reference). Dernyck et al. also describes a MGSA bioassay. Assays for CSF co-factor activity are described in Broxmeyer et al., 1990, Blood, 76:1110 and Broxmeyer et al., 1989, J. Exp. Med., 170:158 (both incorporated herein by reference).

Individual hu-MIP-2β polypeptides serve as agonists or antagonists of one or more of the activities of any of the members of the above-mentioned multigene family. The biological activity assays described above, and receptor binding assays with receptors for MIP-2 multigene family members, are used to screen hu-MIP-2β polypeptides for antagonistic or agonistic activities. Hu-MIP-2β polypeptides exhibiting antagonistic activity will compete with the desired member of the multigene family for binding to a receptor but will also inhibit a biological activity of the desired member, Hu-MIP-2β polypeptides with agonistic activity will possess enhanced biological activity or activities comparable to the desired multigene family member. Some hu-MIP-2β polypeptides with agonistic activity act as co-factors to increase the activity of another multigene family member. Other hu-MIP-2β polypeptides exhibit two or more non-overlapping activities from different members of the multigene family. Some hu-MIP-2β polypeptides act as agonists for one activity and antagonists for another activity, e.g., where one polypeptide inhibits neutrophil activation and increases monocyte activation activity.

As therapeutics, hu-MIP-2β polypeptides with agonistic activity are effective for the same therapeutic applications as hu-MIP-2β, as well as therapeutic applications of other members of the multigene family. Hu-MIP-2β polypeptide with antagonistic activity will suppress malignancy and will prevent inflammatory conditions and autoimmune diseases, such as pathological infiltration of neutrophils, including psoriasis, rheumatoid arthritis, and lung diseases.

An example of a set of hu-MIP-2β polypeptides of interest are the polypeptides which bind to the receptor binding site of IL-8. Hu-MIP-2β can compete effectively with IL-8 for the IL-8 receptor. Hu-MIP-2β polypeptides that are IL-8 agonists will have a three-dimensional structure similar the portion of IL-8 that binds to the receptor. Recent studies of the NMR and X-ray structures for IL-8 are useful for determining the receptor binding site. (Clore et al., 1989, J. Biol. Chem., 264:18907; Clore et al., 1990, Biochem., 29:1689; Clore et al., 1991, J. Mol. Biol., 217:611, and Baldwin et al., 1991, Proc. Natl. Acad. Sci. USA, 88:502).

An amount of hu-MIP-2β polypeptide which is "an effective amount" for the stimulation of myelopoiesis in myelopoietic cells is that amount which produces a detectable increase in myelopoiesis (see, e.g., assay in Broxmeyer, et al., 1989). An effective amount of hu-MIP-2β polypeptide relative to the other activities of such peptides is likewise the amount which produces a detectable response in the appropriate assay as described above.

### Production of Compositions Containing hu-MIP-2β

Compositions containing hu-MIP-2β polypeptides substantially free of other human protein can be prepared by purification from natural sources, by chemical synthesis or by recombinant DNA methods. A crude extract of naturally-produced hu-MIP-2β can be prepared from any convenient source of the native protein. A preferred source is a human macrophage cell line which has been stimulated with LPS. A cell-free extract containing hu-MIP-2β, as determined by, e.g., immunoassay as described below, serves as the stating material for further purification. This purification can be accomplished according to techniques which are well-known in the protein purification art. For example, various types of chromatography may be used. Columns which may be used include a DEAE cellulose column, or an anion exchange column, as well as a gel permeation column. A preferred purification method follows that taught in Wolpe, et al. (1989). The purification process may be monitored by for example immunoassay as described below or by MIP-2 activity assays according to Wolpe, et al. (1989), or Broxmeyer, et al. (1989), which are incorporated herein by reference.

The hu-MIP-2β or peptide fragments thereof can also be purified using immunoaffinity techniques. The use of the antibodies of the present invention to purify the proteins of the invention allows good separation from those proteins which are most similar to them. Of course, other techniques of purification are known in the art and can be used to purify the peptides of the invention.

Alternatively, the hu-MIP-2β polypeptides of this invention may be prepared by chemical synthesis. For example, the Merrifield technique (Journal of American Chemical Society, vol. 85, pp. 2149--2154, 1968), can be used.

It is possible to purify hu-MIP-2β from an appropriate tissue/fluid source; however, it is preferred to produce it by recombinant methods from a DNA sequence encoding hu-MIP-2β, which can be synthesized chemically or isolated by one of several approaches. The DNA sequence to be synthesized can be designed with the appropriate codons for the hu-MIP-2β amino acid sequence. In general, one will select preferred codons for the intended host, if the sequence will be used for expression. The complete sequence is assembled from overlapping oligonucleotides prepared by standard methods and assembled into a complete coding sequence. See, e.g., Edge (1981) Nature 292:756; Nambair, et al. (1984) Science 223:1299; Jay, et al. (1984) J. Biol. Chem., 259:6311. The isolation methods will rely in part on nucleic acid hybridization using appropriate oligonucleotide probes. Such probes can be constructed synthetically, based on the DNA or amino acid sequences disclosed herein, or isolated from genomic or cDNA clones also described herein.

### Preparation of Nucleic Acid Libraries

The basic strategies for preparing oligonucleotide probes and DNA libraries, as well as their screening by nucleic acid hybridization, are well known to those of ordinary skill in the art. See, e.g., "DNA Cloning" Vol. I (D.P. Glover, ed., 1985); "Nucleic Acid Hybridization" (B.D. Hames & S.J. Higgins, eds., 1985); "Oligonucleotide Synthesis" (M.J. Gate, ed., 1984); T. Maniatis, et al., "Molecular Cloning: a Laboratory Manual" (1982); B. Perbal, "A Practical Guide To Molecular Cloning" (1984). First, a DNA library is prepared. The library can consist of a genomic DNA library from a human source. Human genomic libraries are known in the art. See, e.g., Maniatis, et al. (1978) Cell, 15:687-701; Lawn, et al. (1978) Cell, 15:1157-1174. More preferred are DNA libraries constructed of cDNA, prepared from poly-A RNA (mRNA) by reverse transcription. See, e.g., U.S. Patent Nos. 4,446,325; 4,440,859; 4,443,140; 4,431,7400; 4,370,417; 4,363,877. The mRNA is isolated from a cell line or tissue believed to express hu-MIP-2β, such as a macrophage cell line. A suitable source of mRNA for cDNA library constructions is the cell line U937. The genomic DNA or cDNA is cloned into a vector suitable for construction of a library. A preferred vector is a bacteriophage vector, such as any of the phage lambda. The construction of an appropriate library is within the skill of the art. See, e.g., B. Perbal, supra.

### Preparation of Nucleic Acid Probes

Once the library is constructed, oligonucleotides are used to probe the library to identify the segment carrying a sequence encoding hu-MIP-2β polypeptide. In general, the probes are synthesized chemically, preferably based upon known nucleic acid sequences, or alternatively, on predicted amino acid sequences from cDNA clones.

Alternately, in the absence of a good tissue source for the mRNA, it may become necessary to obtain sequences from the protein. The N-terminal sequence can be obtained by N-terminal sequence analysis. Determination of internal sequence can be done, for example, by Staph-V8 proteolysis of protein purified in the usual way, followed by reductive alkylation and separation by HPLC of the digestion products. Elution peaks corresponding to discrete enzyme fragments can then be sequenced by standard methods. From the amino acid sequence, oligonucleotides can be designed and produced for use as hybridization probes to locate either cDNA sequences or the exons in genomic DNA. Ultimately, the isolated exons are ligated together in such a way that they correspond to the nucleic acid sequence which encodes the mature protein.

Nucleotide sequences are selected so as to correspond to the codons encoding the amino acid sequence. Since the genetic code is redundant, it will usually be necessary to synthesize several oligonucleotides to cover all, or a reasonable number, of the possible nucleotide sequences which encode a particular region of the protein. Thus, it is generally preferred, in selecting a region of the sequence upon which to base the probes, that the region not contain amino acids whose codons are highly degenerate. It may not be necessary, however, to prepare probes containing codons whose usage is rare in humans (from which the library was prepared).

Antibodies thereto can be used to immunoprecipitate any of the desired protein present in a selected tissue, cell extract, or body fluid. Purified MIP-2 from this source can then be sequenced and used as a basis for designing specific probes as described above.

One of skill in the art may find it desirable to prepare probes that are fairly long and/or encompass regions of the amino acid sequence which would have a high degree of redundancy in the corresponding nucleic acid sequences. Probes covering the complete gene, or a substantial part of the gene, may also be appropriate, because of the expected degree of homology. The sequence is highly conserved across species lines, and so probes containing the coding sequence from another species, such as the mouse, can be readily used to screen libraries prepared from human DNA. In other cases, it may be desirable to use two sets of probe simultaneously, each to a different region of the gene. While the exact length of any probe employed is not critical, typical probe sequences are no greater than 1000 nucleotides in length, more typically they are not greater than 500 nucleotides, even more typically they are no greater than 250 nucleotides; they may be no greater than 100 nucleotides, and also may be no greater than 75 nucleotides in length. Generally it is recognized in the art that probes from about 14 to about 20 base pairs are usually effective. Because hu-MIP-2β belongs to a group of highly homologous proteins, probes containing sequences unique to hu-MIP-2β are preferred in order to discriminate between the related sequences. Longer probe sequences may be necessary to encompass unique polynucleotide regions with differences sufficient to allow related target sequences to be distinguished. For this reason, probes are preferably from about 10 to about 100 nucleotides in length and more preferably from about 20 to about 50 nucleotides.

### Using Probes to Select Clones

As is known in the art, oligonucleotide probes are labeled with a marker, such as a radionucleotide or biotin, using standard procedures. The labeled set of probes is then used in the screening step, which consists of allowing the single-stranded probe to hybridize to isolated ssDNA from the library, according to standard techniques. Either stringent or permissive hybridization conditions could be appropriate, depending upon several factors including, but not limited to, the length of the probe, whether the probe and library are from the same species, and whether the species are evolutionarily close or distant. It is within the skill of the art to optimize hybridization conditions so that homologous sequences are isolated and detectable above background hybridizations. The basic requirement is that hybridization conditions be of sufficient stringency so that selective hybridization occurs; i.e., hybridization is due to a minimum degree of nucleic acid homology (e.g., at least about 75%), as opposed to non-specific binding or hybridization due to a lower degree of homology. See generally, "Nucleic Acid Hybridization," supra. Because of the number of sequences closely related to mMIP-2, both a unique probe sequence and stringent hybridization conditions are preferred. Once a clone from the screened library has been identified by positive hybridization, it can be further characterized by restriction enzyme analysis and DNA sequencing to confirm that the particular clone contains a coding sequence for the desired protein.

### Genomic Clones

Partial genomic clones can be extended into complete clones by one of several techniques. A clone can be extended in either the 3' or 3' direction using "chromosome walking" techniques to ensure inclusion of the entire gene coding region. Restriction fragments of these clones can then be probed with, for example, cDNA encoding the desired protein. If sufficient homology exists within these exons, other exons could be identified with the same cDNA clone.

Other coding regions in genomic clones may be rapidly identified by direct sequencing of the DNA downstream of a cloned exon using modern M13-dideoxy sequencing techniques. The sequence is then inspected in all three reading frames to reveal an open reading frame. Other exons will also be apparent, since they will be bounded on both sides by intron-splicing signals and should encode amino acids that are common to MIP-2's from different species.

More specifically, once the correct gene coding sequence for at least one exon of hu-MIP-2β is known, it can be used to obtain the entire protein coding region of the enzyme by one or more of the following means. First, the desired sequence fragment can be trimmed from the clone and placed in a more convenient vector, such as pBR322, so that large quantities of DNA containing only the fragment itself can be obtained and used as a hybridization probe. Alternately, a oligonucleotide corresponding to unique regions of the coding region can be synthesized. Either can be used as a hybridization probe for a genomic DNA library.

### cDNA Clones

Mammalian genomic clones (partial or full-length) containing the longest inserts of the gene can be co-transfected into Chinese hamster ovary (CHO) cells with plasmid DNA containing a marker, such as neomycin and metallothionine resistance genes. Surviving cells, selected in the presence of antibiotic G418 and Cd++, can be analyzed for the presence of RNA transcripts which hybridize to the sequence encoding the protein by Northern blot of extracted RNA, Clones containing the desired transcripts can then be used as an mRNA source for a cDNA library construction. Alternatively, northern blots of mRNA obtained from various sources, such as peritoneal cells and pus, endothelial tissue, and peripheral blood leukocytes, lymphocytes, and macrophages can be tested for hybridization to the probe. In addition, mRNA from various cell lines such as LPS-stimulated U937 and HL60 can also be tested. Any tissue or cell source containing detectable levels of hybridizing mRNA is then used to produce a cDNA library which will be screened with the same probes in order to detect a full-length cDNA encoding the desired protein.

### Site-directed Mutagenesis

As mentioned above, a DNA sequence encoding MIP-2 can be prepared synthetically rather than cloned. Synthetic DNA sequences allow convenient construction of genes which will express hu-MIP-2β analogs, particularly "muteins." Alternatively, DNA encoding muteins can be made by site-directed mutagenesis of native MIP-2 genes or cDNAs, and muteins can be made directly using conventional polypeptide synthesis.

Site-directed mutagenesis is preferably conducted by polymerase chain reaction methodology using a primer synthetic oligonucleotide complementary to a single stranded phage DNA comprising the sequence to be mutated, except for limited mismatching representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells which harbor the phage. Theoretically, 50% of the new plaques will contain the phage having, as a single strand, the mutated form; 50% will have the original sequence. The resulting plaques are hybridized with kinased synthetic primer at a temperature which permits hybridization of an exact match, but at which the mis-matches with the original strand are sufficient to prevent hybridization. Plaques which hybridize with the probe are then picked, cultured, and the DNA recovered.

### Cloning for Expression

Once a coding sequence for hu-MIP-2β has been prepared or isolated, it can be cloned into any suitable vector or replicon and thereby maintained in a composition which is substantially free of vectors that do not contain a MIP-2 coding sequence (e.g., free of other clones from the library). Numerous cloning vectors are known to those of skill in the art, and the selection of an appropriate cloning vector is a matter of choice. Examples of recombinant DNA vectors for cloning, and host cells which they can transform, include the various bacteriophage lambda vectors (E. coli), pBR322 (E. coli), pACYC177 (E. coli), pkT230 (gram-negative bacteria), pGV1106 (gram-negative bacteria), pLAFR1 (gram-negative bacteria), pHV14 (E. coli and Bacillus subtilis), pBD9 (Bacillus), pIJ61 (Streptomyces), pUC6 (Streptomyces), actinophage, dC31 (Streptomyces), YIp5 (Saccharomyces), YCp19 (Saccharomyces), and bovine papilloma virus (mammalian cells). See generally, "DNA Cloning," Vols. I & II, supra; T. Maniatis, et al., supra; B. Perbal, supra.

The DNA sequences and DNA molecules of the present invention may be expressed using a wide variety of host/vector combinations. For example, useful vectors may comprise segments of chromosomal, non-chromosomal (such as various known derivatives of SV40 and known bacterial plasmids, e.g., plasmids from E.coli including colEl, pcRl pBR322, pMB9 and RP4), or synthetic DNA sequences, phage DNAs (M13) including derivatives of phage (e.g., NM 989) and filamentous single-stranded DNA phages, vectors useful in yeasts (such as the 2 micron plasmid), vectors useful in eukaryotic cells (such as vectors useful In animal cells, e.g. those containing SV-40 adenovirus and retrovirus derived DNA sequences) and vectors derived from combinations of plasmids and phage DNAs (such as plasmids which have been modified to employ phage DNA), or other derivatives thereof.

According to the present invention, the coding sequence for hu-MIP-2β polypeptide is placed under the control of a promoter, ribosome binding site (for bacterial expression) and, optionally, an operator (collectively referred to herein as "control" elements), so that the DNA sequence encoding hu-MIP-2β polypeptide is transcribed into RNA in the host cell transformed by a vector containing this expression construct. The coding sequence may or may not contain a signal peptide or leader sequence. If the coding sequence contains a signal peptide, it may or may not be the signal sequence naturally associated with hu-MIP-2β. In bacteria for example, mature hu-MIP-2β is preferably made by the expression of a coding sequence which does not contain the mammalian signal peptide, but rather by expression of a coding sequence containing a leader sequence which is removed by the bacterial host in post-translational processing. See e.g., U.S. Patent Nos. 4,431,739; 4,425,437; 4,338,397.

Preferably the expression vector already contains at least one expression control sequence that may be operatively linked to the DNA coding sequence when it is inserted in the vector in order to control and regulate the expression of the cloned DNA sequence. Examples of useful expression control sequences are the lac system, the trp system, the tac system, the trc system, major operator and promoter regions of phage lambda, the control region of fd coat protein, the glycolytic promoters of yeast (e.g., the promoter for 3-phosphoglycerate kinase), the promoters of yeast acid phosphatase (e.g., Pho5), the promoters of the yeast alpha mating factors, and promoters derived from polyoma, adenovirus, retrovirus, or simian virus (e.g., the early and late promoters of SV40), and other sequences known to control the expression of genes of prokaryotic or eukaryotic cells and their viruses or combinations thereof.

An expression vector is constructed according to the present invention so that the hu-MIP-2β coding sequence is located in the vector with the appropriate regulatory sequences such that the coding sequence is transcribed under the "control" of the control sequences (i.e., RNA polymerase which binds to the DNA molecules at the control sequences transcribes the coding sequence). The control sequences may be ligated to the coding sequence prior to insertion into a vector, such as the cloning vectors described above. Alternatively, the coding sequence can be cloned directly into an expression vector which already contains the control sequences and an appropriate restriction site. For expression of the desired protein in procaryotes and yeast, the control sequences will necessarily be heterologous to the coding sequence. If the host cell is a procaryote, it is also necessary that the coding sequence be free of introns (e.g., cDNA). If the selected host cell is a mammalian cell, the control sequences can be heterologous or homologous to the hu-MIP-2β coding sequence, and the coding sequence can either be genomic DNA (containing introns) or cDNA. Either genomic or cDNA coding sequences can be expressed in yeast.

Furthermore, within each specific expression vector, various sites may be selected for insertion of the DNA sequences of this invention. These sites are usually designated by the restriction endonuclease which cuts them. They are well recognized by those of skill in the art. It is, of course, to be understood that an expression vector useful in this invention need not have a restriction endonuclease site for insertion of the chosen DNA fragment. Instead, the vector can be joined to the fragment by alternative means. The expression vector, and in particular the site chosen therein for insertion of a selected DNA fragment and its operative linking therein to an expression control sequence, is determined by a variety of factors, e.g., number of sites susceptible to a particular restriction enzyme, size of the protein and expression characteristics, such as the location of start and stop codons relative to the vector. An insertion site for a DNA sequence is determined by a balance of these factors, not all selections being equally effective for a given case.

A number of procaryotic expression vectors are known in the art. See, e.g., U.S. Patent Nos. 4,440,859; 4,436,815; 4,431,740; 4,431,739; 4,428,941; 4,425,437; 4,418,149; 4,411,994; 4,366,246; 4,342,832; see also U.K. Pub. Nos. GB 2,121,054; GB 2,008,123; GB 2,007,675; and European Pub. No. 103,395. Preferred procaryotic expression systems are in E. coli. Other preferred expression vectors are those for use in eucaryotic systems. A preferred eucaryotic expression system is that employing vaccinia virus, which is well-known in the art. See, e.g., Mackett, et al. (1984) J. Virol. 49:857; "DNA Cloning." vol. II, pp. 191-211, supra; PCT Pub. No. WO 86/07593. Yeast expression vectors are known in the art. See, e.g., U.S. Patent Nos. 4,446,235; 4,443,539; 4,430,428; see also European Pub. Nos. 103,409; 100,561; 96,491. Another expression system is vector pHS1, which transforms Chinese hamster ovary cells. The use of the vector is described in PCT Pub. No. WO 87/02062.

Useful expression hosts may include well known eukaryotic and prokaryotic hosts, such as strains of E. coli, such as E.coli SG-936, E.coli HB 101, E.coli w3110, E.coli X1776, E.coli X2282, E.coli DHI, and E.coli MRC1, Pseudomonas, Bacillus, such as Bacillus subtilis, Streptomyces, yeasts and other fungi, animal cells, such as COS cells and CHO cells, and human cells and plant cells in tissue culture.

Of course, not all host/expression vector combinations function with equal efficiency in expressing the DNA sequences of this invention or in producing the polypeptides of this invention. However, a particular selection of a host/expression vector combination may be made by those skilled in the art. For example, the selection should be based on a balancing of a number of factors. These include compatibility of the host and vector, toxicity of the proteins encoded by the DNA sequence to the host, ease of recovery of the desired protein, expression characteristics of the DNA sequences and the expression control sequences operatively linked to them, biosafety, costs and the folding, form or any other necessary post-expression modifications of the desired protein. Preferably, the host cell will not express proteases which degrade hu-MIP-2β.

### Cloning in a Yeast Expression System

A preferred expression system is yeast. Hu-MIP-2β can be expressed by a yeast cell transformed with an expression vector containing DNA coding sequence for hu-MIP-2β under the control of a yeast promoter. Such expression vectors may be constructed as follows.

A yeast promoter is any DNA sequence capable of binding yeast RNA polymerase and initiating the downstream (3') transcription of a coding sequence (e.g., structural gene) into mRNA. A promoter will have a transcription initiation region which is usually placed proximal to the 5' end of the coding sequence. This transcription initiation region typically includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (E.P.O Pub. No. 284044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK)(E.P.O. Pub. No. 329203). The yeast PHO5 gene, encoding acid phosphatase, also provides useful promoter sequences [Miyanohara, et al., (1983) Proc. Natl. Acad. Sci. USA, 80:1].

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (U.S. Patent Nos. 4,876,197; 4,880,734). Other examples of hybrid promoters include promoters which consist of the regulatory sequences of either the ADH2, GAL4, GAL10, or PHO5 genes, combined with the transcriptional activation region of a glycolytic enzyme gene such as GAP or PyK (E.P.O. Pub. No. 164556). Furthermore, a yeast promoter can include naturally occurring promoters of non-yeast origin that have the ability to bind yeast RNA polymerase and initiate transcription. See, e.g., Cohen, et al. (1980) Proc. Natl. Acad. Sci. USA, 77:1078; Henikoff, et al., (1981) Nature, 283:835; Hollenberg, et al., (1981) Curr. Topics Microbiol. Immunol., 96:119; Hollenberg, et al., "The Expression of Bacterial Antibiotic Resistance Genes in the Yeast Saccharomyces cerevisiae," in: Plasmids of Medical Environmental and Commercial Importance (eds., K.N. Timmis and A. Puhler); Mercereau-Puigalon, et al. (1980) Gene 11:163; Panthier, et al. (1980), Curr. Genet., 2:109.

A DNA molecule may be expressed intracellularly. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by in vitro incubation with cyanogen bromide.

Fusion proteins provide an alternative to direct expression. Typically, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See, e.g., E.P.O. Pub. No. 196056. Another example is a ubiquitin fusion protein. Such a fusion protein is made with the ubiquitin "leader" or "pro-" region that preferably retains a site for a processing enzyme (e.g. ubiquitin-specific processing protease) to cleave the ubiquitin from the foreign protein. Through this method, therefore, native foreign protein can be isolated (P.C.T. WO 88/024066; commonly owned U.S. Patent Application Serial No. 390,599, filed 7 August 1989, or foreign patents or applications claiming priority therefrom (as listed in World Patents Index produced by Derwent Publications, Ltd.), the disclosure of which is incorporated herein by reference).

Alternatively foreign proteins can also be secreted from the cell into the growth media by creating chimeric DNA molecules that encode a fusion protein comprised of a leader sequence fragment that provide for secretion in yeast and the foreign gene. Preferably, there are processing sites (in vivo or in vitro) encoded between the leader fragment and the foreign gene. The leader sequence fragment typically encodes a signal peptide comprised of hydrophobic amino acids which direct the secretion of the protein from the cell.

DNA encoding suitable signal sequences can be derived from genes for secreted yeast proteins, such as the yeast invertase gene (E.P.O. Pub. No. 12,873; J.P.O. Pub. No. 62,096,086) and the A-factor gene (U.S. Patent No. 4,588,684). Alternatively, leaders of non-yeast origin, such as an interferon leader, exist that also provide for secretion in yeast (E.P.O. Pub. No. 60057).

A preferred class of secretion leaders are those that employ a fragment of the yeast α-factor gene, which contains both a "pre" signal sequence, and a "pro" region. The types of α-factor fragments that can be employed include the full-length pre-pro α-factor leader (about 83 amino acid residues) as well as truncated α-factor leaders (typically about 25 to about 50 amino acid residues) (U.S. Patent Nos. 4,546,083 and 4,870,008; E.P.O. Pub. No. 324274). Additional leaders employing an α-factor leader fragment that provides for secretion include hybrid α-factor leaders made with a pre-sequence of a first yeast, but a pro-region from a second yeast α-factor. (See, e.g., P.C.T. WO 89/02463.)

Typically, transcription termination sequences recognized by yeast are regulatory regions located 3' to the translation stop codon, and thus, together with the promoter, flank the coding sequence. These sequences direct the transcription of an mRNA which can be translated into the polypeptide encoded by the DNA. Examples of transcription terminator sequence include the wild-type α-factor transcription termination sequence and other yeast-recognized termination sequences, such as those for glycolytic enzymes.

Typically the above described components, comprising a promoter, leader (if desired), coding sequence of interest, and transcription termination sequence, are put together into expression constructs. Expression constructs are often maintained in a replicon, such as an extrachromosomal element (e.g., plasmids) capable of stable maintenance in a host, such as yeast or bacteria. The replicon may have two replication systems, thus allowing it to be maintained, for example, in yeast for expression and in a procaryotic host for cloning and amplification. Examples of such yeast-bacteria shuttle vectors include YEp24 [Botstein, et al., (1979) Gene 8:17-24], pCl/1 [Brake, et al., (1984), Proc. Natl. Acad. Sci. USA, 81:4642-4646], and YRp17 [Stinchcomb, et al., (1982), J. Mol. Biol., 158:157]. In addition, a replicon may be either a high or low copy number plasmid. A high copy number plasmid will generally have a copy number ranging from about 5 to about 200, and typically about 10 to about 150. A host containing a high copy number plasmid will preferably have at least about 10, and more preferably at least about 20. Either a high or low copy number vector may be selected, depending upon the effect of the vector and the foreign protein on the host. See, e.g., Brake, et al., supra.

Alternatively, the expression constructs can be integrated into the yeast genome with an integrating vector. Integrating vectors typically contain at least one sequence homologous to a yeast chromosome that allows the vector to integrate, and preferably contain two homologous sequences flanking the expression construct. Integrations appear to result from recombinations between homologous DNA in the vector and the yeast chromosome (Orr-Weaver, et al. 1983), Methods in Enzymol, 101:228-245). An integrating vector may be directed to a specific locus in yeast by selecting the appropriate homologous sequence for inclusion in the vector. See Orr-Weaver, et al., supra. One or more expression constructs may integrate, possibly affecting levels of recombinant protein produced [Rine, et al., (1983), Proc. Natl. Acad. Sci. USA, 80:6750]. The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Typically, extrachromosomal and integrating expression constructs may contain selectable markers to allow for the selection of yeast strains that have been transformed. Selectable markers may include biosynthetic genes such as ADE2, HIS4, LEU2, TRP1, and URA3. Selectable markers may also include drug resistance genes such as ALG7 and the G418 resistance gene, which confer resistance in yeast cells to tunicamycin and G418, respectively. In addition, a suitable selectable marker may also provide yeast with the ability to grow in the presence of toxic compounds, such as metal. For example, the presence of CUP1 allows yeast to grow in the presence of copper ions [Butt, et al. (1987), Microbiol. Rev., 51:351].

Expression vectors, either extrachromosomal replicons or integrating vectors, have been developed for transformation into many yeasts. For example, expression vectors have been developed for, inter alia, the following yeasts: Candida albicans [Kurtz, et al., (1986), Mol. Cell Biol., 6:142], Candida maltosa [Kunze, et al., (1985), J. Basic Microbiol., 25:141], Hansenula polymorpha [Gleeson, et al., (1986), J. Gen. Microbiol., 132:3459; Roggenkamp, et al. (1986), Mol. Gen. Genet., 202:302], Kluyveromyces fragilis [Das, et al., (1984), J. Bacteriol., 158:1165], Kluyveromycces lactis [De Louvencourt et al., (1983), J. Bacteriol., 154:737; Van den Berg, et al., (1990) Bio/Technology, 8:135], Pichia guillerimondii [Kunze et al., (1985), J. Basic Microbiol., 25:141], Pichia pastoris [Cregg, et al., (1985), Mol. Cell Biol., 5:3376; U.S. Patent Nos. 4,837,148 and 4,929,555], Saccharomyces cerevisiae [Hinnen et al., (1978), Proc. Natl. Acad. Sci. USA, 75:1929; Ito, et al., (1983) J. Bacteriol., 153:163], Schizosaccharomyces pombe [Beach and Nurse (1981), Nature, 300:706], and Yarrowia lipolytica [Davidow, et al., (1985), Curr. Genet., 10:39-48; Gaillardin, et al. (1985), Curr. Genet., 10:49].

Methods of introducing exogenous DNA into yeast hosts are well-known in the art, and typically include either the transformation of spheroplasts or of intact yeast cells treated with alkali cations. Transformation procedures usually vary with the yeast species to be transformed. See, e.g., Kurtz, et al. (1986), Mol. Cell. Biol., 6:142, Kunze, et al. (1985), J. Basic Microbiol., 25:141, for Candida; Gleeson, et al., (1986), J. Gen. Microbiol., 132:3459, Roggenkamp, et al. (1986), Mol. Gen. Genet., 202:302, for Hansenula; Das, et al., (1984), J. Bacteriol., 158:1165, De Louvencourt et al., (1983), J. Bacteriol., 154:1165, Van den Berg, et al., (1990), Bio/Technology. 8:135, for Kluyveromyces; Cregg. et al., (1985), Mol. Cell Biol, 5:3376, Kunze, et al. (1985), J. Basic Microbiol., 25:141, U.S. Patent Nos. 4,837,148 and 4,929,555, for Pichia; Hinnen, et al. (1978), Proc. Natl. Acad. Sci. USA, 75:1929, Ito, et al. (1983), J Bacteriol., 153:163, for Saccharomyces; Davidow, et al., (1985) Curr. Genet., 10:39, Gaillardin, et al. (1985), Curr. Genet., 10:49, for Yarrowia.

### Expression of the Protein

Depending on the expression system and host selected, the protein is produced by growing host cells transformed by an expression vector containing the coding sequence for hu-MIP-2β under conditions whereby the protein is expressed. The protein is then isolated from the host cells and purified. The selection of the appropriate growth conditions and recovery methods are within the skill of the art.

One preferred means of obtaining the preparations of this invention is to culture cells transfected with an expression vector comprising the intron-free DNA corresponding to hu-MIP-2β, using appropriate culture conditions. The cells are then harvested and the cell fraction may be separated by standard separation procedures, such as centrifugation. If the expression system secretes the polypeptide into growth media, the polypeptide can be purified directly from cell-free media. If the protein is not secreted, it is isolated from cell lysates. Once a crude extract containing hu-MIP-2β is obtained, purification can be accomplished as described above, using standard techniques.

In general, recombinant production of hu-MIP-2β polypeptide can provide compositions of this cytokine substantially free of other human proteins. The ability to obtain high levels of purity is one result of using recombinant expression systems which also can produce hu-MIP-2β polypeptides in substantial quantities vis-a-vis in vivo sources. Thus, by applying conventional techniques to recombinant cultures, hu-MIP-2β polypeptide compositions can be produced more readily. Those of ordinary skill in the art can select among the above techniques to prepare the substantially pure peptides of this invention.

### Pools of hu-HIP-2β Analogs

Compositions containing pools of hu-MIP-2β analogs substantially free of other human proteins can also be prepared, preferably by recombinant DNA methods or chemical synthesis. These pools can be screened using a receptor binding assay or any of the biological activity assays described above for the hu-MIP-2β analogs with the desired activities.

For example, Parmley and Smith, 1988, Gene, 73:305 (incorporated herein by reference), describe a protocol for producing and screening pools of proteins using recombinant DNA methods. This protocol, with a receptor to a MIP-2 multigene family member, can be used to screen pools of hu-MIP-2β muteins, truncated hu-MIP-2β peptides, or hu-MIP-2β fusion proteins. First, pools of DNA fragments encoding different hu-MIP-2β analogs can be made. Next, the pool of DNA fragments can be inserted into the genome of the filametnous phage described in Parmley and Smith. This library of phages will produce colonies, whose expression products can be screened. The DNA from the desired colonies can be isolated and used to identify the desired hu-MIP-2β analog(s).

Pools of hu-MIP-2β analogs can also be synthesized. For convenience in a receptor binding assay, hu-MIP-2β analogs can be synthesized on polyethylene pins arranged in a 96-pin array on a block (matching the spacing of a standard 96-well microtiter plate). See for example Geyesen, U.S. Patent No. 4,708,871 (incorporated herein by reference in full), which describes such a process of synthesizing polypeptides applied to the VP1 protein of foot and mount disease virus. Other methods for producing libraries of polypeptides and selecting the polypeptides with specific properties are described in U.S. Patent No. 5,010,175 (incorporated herein by reference in full), and U.S. Patent Application No. 07/652,194 filed 6 February 1991 (incorporated herein by reference), or foreign patents or applications claiming priority therefrom (as listed in World Patents Index produced by Derwent Publishing Ltd.).

Pools of polypeptides unrelated to the hu-MIP-2β can be produced, using the recombinant DNA and chemical synthesis methods above, and screened for their ability to bind receptors for members of the MIP-2 gene family. Next, the polypeptides that do bind the receptor(s) can then be assayed in the biological activity assays described above to determine their agonistic or antagonistic activities. Such polypeptides are effective for the same therapeutic applications as hu-MIP-2α polypeptides. Another method of screening pools of peptides is described in Cwirla et al., 1990, Proc. Natl. Acad. Sci. USA, 87:6378 (incorporated herein by reference).

For ease of pharmaceutical administration, small organic molecules may be preferred over these polypeptide agonists and antagonists. From the structures of the polypeptides, small organic molecules that mimic the shape and activities of these polypeptides may be found. Methods of determining the structures of these hu-MIP-2β agonists and antagonists are known in the art and include x-ray crystallography and 2-dimensional nuclear magnetic resonance.

### Production of Antibodies

Native, recombinant or synthetic hu-MIP-2β polypeptide (full length or fragments containing one or more epitopes) can be used to produce both polyclonal and monoclonal antibodies. If polyclonal antibodies are desired, hu-MIP-2β polypeptide is used to immunize a selected mammal (e.g., mouse, rabbit, goat, horse, etc.) and serum from the immunized animal later collected and treated according to known procedures. The hu-MIP-2β polypeptide of the present invention can be used to stimulate production of antibodies in a mammal by immunizing the mammal with the preparation. Such immunization may optionally employ coupling of the polypeptide to a larger immunogenic substance such as keyhole limpet hemocyanin. Immunization of mammals, such as rabbits, mice, goats, etc., to produce antibodies is well known in the art.

Polyclonal antibody preparations can be partially purified using immunoaffinity techniques employing a synthetic or recombinant polypeptide of the present invention. Such purification methods are well-known in the art. In particular, compositions containing polyclonal antibodies to a variety of antigens in addition to the desired protein can be made substantially free of antibodies which are directed to other antigens by immunoaffinity chromatography. The substantially pure preparation of polypeptide of the present invention can be used to affinity purify antibodies reactive with hu-MIP-2β polypeptide. For affinity purification of antibodies, the polypeptide can be coupled to an inert matrix, such as agarose beads. Techniques for such coupling are well known in the art.

A preparation of the polypeptide can also be used to detect or quantitate antibodies specific for hu-MIP-2β in an antibody preparation or other biological sample. In such a case, the synthetic peptide will usually be coupled to a larger substance, such as bovine serum albumin or a solid support. Once again, the techniques for coupling polypeptides to such matrices are well known in the art.

### Monoclonal Antibodies

Monoclonal antibodies reactive with hu-MIP-2β are also readily produced by one skilled in the art following the disclosure herein. The general methodology for making monoclonal antibodies by hybridomas is well known. Monoclonal antibodies can be raised which are reactive with unique hu-MIP-2β epitopes. Generally, a rat or mouse is immunized with the polypeptide of the present invention, and the rodent will later be sacrificed and spleen cells recovered for fusion with myeloma cells. Hybrid cells can be selected according to techniques known In the art. Antibody production of each hybrid cell can be screened individually to select antibodies which bind to epitopes on hu-MIP-2β.

In order to screen for antibodies which are immunoreactive with epitopes unique to hu-MIP-2β polypeptides, a simple battery of tests can be performed. Antibodies can be tested for immunoreactivity with hu-MIP-2β polypeptide using a substantially pure preparation of the hu-MIP-2β polypeptide of the present invention. The desired specific antibodies should be positive in this test. The antibody can then be tested for immunoreactivity with other members of the MIP-2 multigene family. Generally, antibodies which are negative in this test react with unique hu-MIP-2β epitopes.

Immortal, antibody-producing cell lines can also be created by techniques other than fusion, such as direct transformation of B lymphocytes with oncogenic DNA, or transfection with Epstein-Barr virus. See, e.g., M. Schreier, et al., "Hybridoma Techniques" (1980); Hammerling, et al., "Monoclonal Antibodies and T-Cell Hybridomas" (1981); Kennett, et al., "Monoclonal Antibodies" (1980); see also U.S. Patent Nos. 4,341,761; 4,399,121; 4,427,783; 4,444,887; 4,451,570; 4,466,917; 4,472,500; 4,491,632; 4,493,890.

Panels of monoclonal antibodies produced against hu-MIP-2β can be screened for various properties; i.e., isotype, epitope, affinity, etc. Of particular interest are monoclonal antibodies that neutralize the activity of MIP-2 or hu-MIP-2β peptides. Such monoclonals can be readily identified using MIP-2 activity assays such as those taught in Wolpe, et al. (1989) and Broxmeyer, et al. (1989) which are incorporated herein by reference. High affinity antibodies are also useful in immunoaffinity purification of native or recombinant hu-MIP-2β.

The types of antibodies contemplated by the present invention include, vertebrate antibodies, particularly mammalian (polyclonal and monoclonal), hybrid antibodies, chimeric antibodies, humanized antibodies, altered antibodies, univalent antibodies, single domain antibodies, as well as functional antibody fragments, such as Fab proteins, so long as they bind selectively to an epitope. For a general review see Winter & Milstein, 1991, Nature, 349:293; Riechmann et al. 1988, Nature, 332:323; Ward et al., 1989, Nature 341:544; U.S. Patent No. 4,816,467; and Glennie et al. 1982, Nature 295:712.

Epitopes are antigenic determinants of a polypeptide. An epitope could comprise 3 or more amino acids in a spatial conformation unique to the epitope. Generally, an epitope consists of at least 5 such amino acids and, more usually, corsets of at least 8-10 such amino acids. Methods of determining spatial conformation of amino acids are known in the art and include, for example, x-ray crystallography and 2-dimensional nuclear magnetic resonance.

### Diagnostic Assays for hu-MIP-2β

Detection of hu-MIP-2β may be on the nucleotide or peptide level. Antibodies can be prepared by immunizing mammals with peptides expressed from the sequences corresponding to hu-MIP-2β polypeptides, as indicated above, and selecting those antibodies specific to hu-MIP-2β using techniques that are well known to those skilled in the art. The particular procedures for gene probe assays and immunoassays will be well-known to those skilled in the art.

### Immunoassay Methods

Antibodies are useful in diagnostic applications. Antibody specific for hu-MIP-2β can be formulated into any conventional immunoassay format; e.g., homogeneous or heterogeneous, radioimmunoassay or ELISA. The various formats are well known in those skilled in the art. See, e.g., "Immunoassay: A Practical Guide" (D.W. Chan and M.T. Perlstein eds. 1987) the disclosure of which is incorporated herein by reference.

Antibodies of the invention are capable of binding to hu-MIP-2β, and preferably they will not bind to hu-MIP-2α. These antibodies also permit the use of imaging analysis with isotopes, conjugated antibodies, or other ligands. One example of a suitable imaging material is ¹²⁵I conjugated to the antibodies specific for hu-MIP-2β.

The antibodies of the present invention can be used to detect hu-MIP-2β epitopes in histological sections of tissue as well as in serum. One can detect antibody binding to tissue sections by any detection means known in the art, for example, radioimmunoassay, enzyme linked immunoadsorbent assay, complement fixation, nephelometric assay, immunodiffusion, immunoelectrophoretic assay and the like.

A particularly useful stain employs peroxidase, hydrogen peroxide and a chromogenic substance such as aminoethyl carbazole. The peroxidase (a well known enzyme, available from many sources) can be coupled to the antibody specific for hu-MIP-2β or merely complexed to it via one or more antibodies. Other chromogenic substances and enzymes may also be used. Such techniques are well known in the art. Radio-labeled antibodies may be specific for hu-MIP-2β or may be second antibodies immunoreactive with antibodies specific for hu-MIP-2β. Again, such techniques are well known. The precise technique by which hu-MIP-2β is detected is not critical to the invention.

One particularly preferred method of detecting and/or quantitating hu-MIP-2β in biological samples employs a competitive assay. An antibody immunoreactive with an epitope found on hu-MIP-2β but not found on hu-MIP-2α is attached to a solid support such as a polystyrene microtiter dish or nitrocellulose paper, using techniques known in the art. The solid support is then incubated in the presence of the sample to be analyzed under conditions where antibody-antigen complexes form and are stable. Excess and unbound components of the sample are removed, and the solid support is washed so that antibody-antigen complexes are retained on the solid support. A fixed amount of a labelled polypeptide, containing an epitope found on hu-MIP-2β but not found on hu-MIP-2α, is then incubated with the solid support. The labelled polypeptide has been coupled to a detectable moiety, such as biotin, peroxidase or radiolabel, by means well known in the art. The labelled polypeptide binds to an antibody immunoreactive with hu-MIP-2β which is attached to the solid support. Excess and unbound polypeptide is removed and the solid support is washed, as above. The detectable moiety attached to the solid support is quantitated. Since the hu-MIP-2β and the polypeptide have competed for the same antibody binding sites, the hu-MIP-2β in the sample can be quantitated by its diminution of the binding of the polypeptide to the solid support. Alternatively, the sample and the labelled polypeptide may be incubated with the solid support at the same time.

### Nucleotide Probe Assays

The nucleotide sequences provided by the invention can be used to form gene probes in accordance with any of the standard techniques. The size of a probe can vary from less than approximately 20 nucleotides to hundreds of nucleotides. The probe may be radio-labeled, labeled with a fluorescent material, or the like. Procedures for the preparation and labeling of nucleotide probes are well known in the art.

The diagnostic test employing a nucleotide probe will employ a biological sample from an individual. Nucleic acids are recovered from the sample employing standard techniques well known to those skilled in the art. The nucleic acid then is incubated with the probe and hybridization is thereafter detected.

Described below are examples of the present invention which are provided only for illustrative purposes. They are not intended to limit the scope of the present invention in any way, as numerous embodiments within the scope of the claims will be apparent to those of ordinary skill in the art in light of the present disclosure. Those of ordinary skill in the art are presumed to be familiar with (or to have ready access to) the references cited in the application, and the disclosures thereof are incorporated by reference herein.

### Example 1

### Isolation and Cloning of cDNA Encoding Murine MIP-2

### cDNA Library Construction

The isolation of poly-A+ RNA from E. coli LPS-stimulated murine RAW 264.7 cells and the construction of a cDNA library have been described previously (Davatelis, et al., 1988).

### Murine MIP-2 cDNA Isolation

A degenerate oligonucleotide probe pool corresponding to amino acids 9-14 of the NH₂-terminal sequence of MIP-2 (Wolpe, et al., 1989) was synthesized. This portion of the partial sequence was chosen because it was predicted to be in a highly conserved coding region and because of its lower codon degeneracy when compared to the other parts of the partial sequence. The resulting probe was a 128-fold degenerate pool of oligomers 17 nucleotides in length.

Duplicate nitrocellulose filter lifts of the plated RAW 264.7 cDNA library (5x10⁵ plaques) were prehybridized at 42°C in 5xSSC, 2x Denhardt's, 50 mM sodium phosphate buffer, pH 6.5, 50% formamide, 0.2% SDS and 0.25 mg/ml sonicated salmon sperm DNA and then were hybridized overnight at 42°C in 5xSSC, 1x Denhardt's, 20 mM sodium phosphate buffer, pH 6.5, 50% formamide, 10% dextran sulfate, 0.1% SDS, 0.1 mg/ml sonicated salmon sperm DNA and 5x10⁴ cpm per ml per degeneracy of ³²P-ATP 5' end-labelled synthetic oligonucleotide probe pool. Following hybridization the filters were washed employing TMAC (Wood, et al., 1985, Proc. Natl. Acad. Sci. USA, 82:1585). Plaques that were positive on duplicate filters were subjected to a second round of low density plating and screening. Positive phage clones were isolated from which DNA was prepared for further analysis.

### Cloning of Murine MIP-2 cDNA

Screening of the cDNA library derived from poly-A+ RNA from RAW 264.7 cells with a degenerate oligonucleotide pool specific for the N-terminal sequence of murine MIP-2 (Wolpe, et al., 1989) resulted in the Isolation of clone MIP-2-20α. Insert cDNA (approx. 1100 bp) was Isolated, cloned into MI3 and the nucleotide sequence determined. The nucleotide sequence and predicted protein sequence are shown in Figure 1. The predicted mature protein sequence starting at position 1 exactly matches the N-terminal peptide sequence determined previously for purified MIP-2 (Wolpe, et al., 1989).

### Example 2

### Isolation and Cloning of cDNA Encoding Human MIP-2α and β

In order to isolate the human homolog(s) of murine MIP-2 cDNA, a fragment encoding most of the mature mMIP-2 protein was isolated and used to probe a U937 cDNA library prepared from poly-A+ RNA of PMA-treated and LPS-stimulated cells. DNA from plaques positive on low stringency wash was isolated and subjected to restriction endonuclease analysis which suggested the presence of two classes of clones. Insert cDNA from representative clones of each class was subcloned into M13 and the nucleotide sequences were determined.

### cDNA Library Construction

The stimulation of the human monocytic-like cell line U937 (Sundstrom, et al., 1976, Inc. J. Cancer, 17:565), the isolation of total and poly-A+ RNA and the construction of a cDNA library were performed as follows. U937 cells (American Type Culture Collection, Rockville, MD) were grown to confluence and stimulated to differentiate by the addition of PMA to a final concentration of 5x10⁸M. After 24 hours in the presence of PMA, LPS (LPS W, E. coli 0127:B8; Difco Laboratories, Inc., Detroit, MI) was added to a final concentration of 1»g/ml and the cells were incubated for an additional 3 hours at 37°C. Total RNA was prepared essentially as described (Cathala, et al., 1983, DNA, 2:329). Poly-A+ RNA was prepared by a single passage over oligo dT cellulose essentially as described (Maniantis, et al., 1982). Double-stranded cDNA was prepared using a kit for cDNA synthesis (Pharmacia LKB Biotechnology, Inc., Pleasant Hill, CA) and cloned and packaged into λgt10.

### Isolation of human homologs of murine MIP-2

Plating of the U937 cDNA library, nitrocellulose filter prehybridization and hybridization of the filters were performed as described in Example 1 for the screening of the RAW 264.7 cDNA library. The probe DNA was a 186 bp Bal I-BglII fragment isolated from the mMIP-2 cDNA. The BglII site was introduced by in vitro mutagenesis using the mutagenic primer 5'-CAAAAGATCTTGAACAAAG-3'. The BalI-BglII fragment encodes most of the mature mMIP-2 amino acid sequence, lacking those base pairs encoding the 3 N-terminal and 8 C-terminal amino acids. This fragment was nick-translated and approximately 500,000 cpm per ml included in the hybridization solution.

After hybridization, filters were subjected to three low stringency washes at room temperature for 30 minutes each in 2xSSC, 0.1% SDS. Plaques positive on duplicate filters were subjected to a second round of low density plating and screening. Positive phage clones were isolated from which DNA was prepared for further analysis.

### Cloning and DNA Sequence Analysis

cDNA inserts were subcloned into M13 phage vectors and DNA sequencing was performed by the dideoxy chain termination method of Sanger et al. (1977, Proc. Natl. Acad. Sci. USA, 74:5463).

The nucleotide sequence and predicted amino acid sequence of hu-MIP-2α is presented in Figure 2. This sequence was confirmed on four independent clones and is representative of the more abundant of the two classes of human cDNA homologous to mMIP-2. The nucleotide sequence and predicted amino acid sequence of hu-MIP-2β, representative of a second class of human cDNAs homologous to mMIP-2 is shown in Figure 3. This sequence was confirmed on two independent clones.

### Example 3

### Restriction Fragment Analysis

Genomic DNA from RAW 264.7 cells was isolated as described by DiLella, et al. (1987, in "Guide to Molecular Cloning Techniques," Berger, et al, eds., Academic Press, Orlando, p. 199). Human genomic DNA and murine C3H/HeN genomic DNA were purchased from Clontech (Palo Alto, CA).

Genomic DNA was digested with restriction enzymes according to the supplier's specifications. Digested DNA was separated on 1% agarose gels and then transferred to HyBond nylon membranes (Amersham, Arlington Heights, IL). Filters were prehybridized and hybridized in 50 mM sodium phosphate, pH 6.5, 5xSSC, 1 mM sodium pyrophospate, 40% formamide, 10% dextran sulfate, 5x Denhardt's solution, 0.1% SDS and 100 mg/ml sonicated salmon sperm DNA. DNAs used for Southern analysis were the 1.1 kb mMIP-2 cDNA (clone mMIP-2-20a), the 0.98 kb hu-MIP-2β cDNA (clone hu-MIP-2-4a) and a 1.05 kb hu-MIP-2α cDNA (clone hu-MIP-2-5a). All cDNAs were labelled by random priming with ³²P-CTP employing a Multiprimer DNA Labelling System (Amersham, Arlington Heights, IL). Following prehybridization for 2-4 hours at 37°C, labelled cDNA was added at 1x10⁶ cpm per ml. Hybridization was for 16-18 hr at 37°C. Filters were rinsed at room temperature for 10 min in 2xSSC, 0.1% SDS, then washed 3 times at 65°C for 45 min each in 0.1xSSC, 0.1% SDS. In some cases hybridized probe was stripped from the blot by treatment for 45 min at 65°C in 0.5xSSC, 0.1% SDS and 50% formamide to allow re-hybridization.

### Southern Analysis

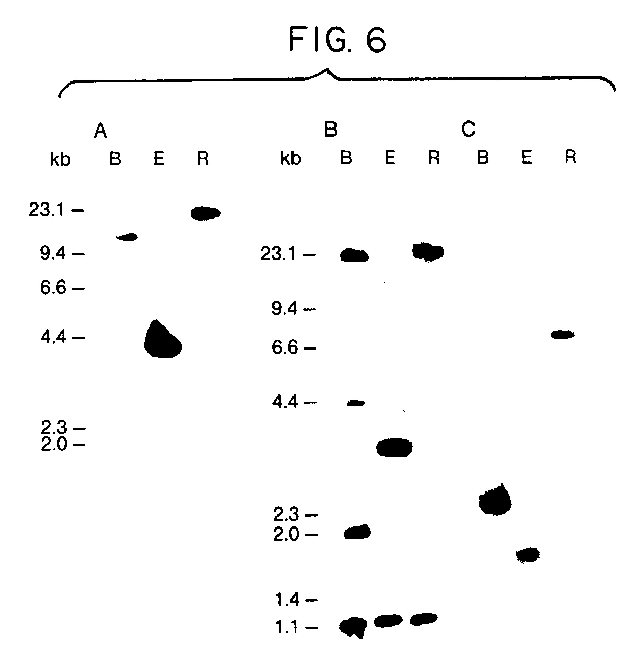

RAW 264.7 DNA was digested with each of three restriction enzymes, BamH1, EcoR1 and EcoRV, separated by agarose gel electrophoresis and probed with ³²P-labelled mMIP-2 cDNA. The results, shown in Figure 6A are consistent with mMIP-2 cDNA defining a single gene. The same results were obtained when mouse C3N/HeJ DNA was similarly analyzed (date not shown).

A Southern analysis of human genomic DNA was performed with hu-MIP-2α and hu-MIP-2β cDNA probes. Hybridization and wash conditions were determined that made it possible to distinguish between hu-MIP-2α and hu-MIP-2β when probed with their respective cDNAs. The conditions used, however, do not distinguish between hu-MIP-2α and hu-gro/MGSA specific sequences. The results of Southern analysis of genomic human DNA restricted with BamH1, EcoR1, or EcoRV and probed with hu-MIP-2α or hu-MIP-2β cDNA are presented in Figures 6B and C, respectively. The patterns of hybridization obtained with each cDNA clearly differ. MIP-2α cDNA hybridized strongly to EcoRV fragments of approximately 23 and 1.1 kb, whereas MIP-2β cDNA hybridized to a 7.0 kb EcoRV fragment. Similarly, hu-MIP-2β cDNA hybridized to 1.8 kb and 3.3 kb (weakly) EcoR1 fragments whereas hu-MIP-2α hybridized strongly to 3.3 and an approximately 1.1 kb EcoR1 DNA fragments and weakly to 4.6 and 3.8 kb EcoR1 fragments. Finally, MIP-2β cDNA hybridizes strongly to a 2.4 kb BamH1 fragment and very weakly to a 4.3 kb BamH1 fragment, whereas human MIP-2α cDNA hybridizes strongly to 20, 2.0 and 1.1 kb BamH1 fragments and less strongly to a 4.3 kb BamH1 fragment. The greater complexity of the hybridization patterns obtained with hu-MIP-2α cDNA probe, especially from BamH1 digested DNA, relative to that obtained with hu-MIP-2β cDNA probe, suggest that the hu-MIP-2α probe is detecting more than one gene, presumably hu-gro as well as hu-MIP-2α. We can conclude from the data shown here that hu-MIP-2β and hu-MIP-2α are two distinct genes.

### Example 4

### Cloning and Expression in Prokaryotes

Plasmid ARV-2 p25 gag (deposited with the American Type Culture Collection, Rockville, Maryland, on 27 August 1985, accession no. 53246) is a derivative of pBR322 containing the tac promoter, Shine Delgarno sequences, and a polylinker as a substitution of the original pBR322 sequences comprised between the EcoRI and PvuII restriction sties. See also EP 181,150.

Clone hu-MIP-2-4a (Example 3) is cleaved with PvuII and BalI, and ligated with a linker having the sequence: 
AATTATGGCGCCCCTGG
TACCGCGGGGACC
The plasmid ARV-2 p25 gag is then cleaved in EcoRI and PvuII, and the ligated hu-MIP-2-4a inserted. The resulting construct is then transformed into competent E. coli D1210 cells, following the protocol of Cohen, et al., Proc. Natl. Acad. Sci. USA (1972) 62:2110). The cells are transformed with 25-50 ng of the construct, and the transformation mix plated on agar plates made in L-broth containing 100 »g/mL ampicillin. Plates are incubated at 37°C for 12 hours, and ampicillin-resistant colonies transferred into 1 mL L-broth containing 100 »g/mL ampicillin. Cells are grown at 37°C, and expression induced by adding 10 »L of 100 mM IPTG to a final concentration of 1 mM, followed by incubation at 37°C for 2 hours. The cells are then lysed, and the product purified.

### Example 5

### Expression in Yeast

Plasmid pGAI1 contains DNA encoding the yeast α-factor leader, a gene for human proinsulin, and a α-factor terminator, under transcriptional control of the yeast glyceraldehyde 3-phosphate dehydrogenase (GAPDH) promoter. The vector is fully described in EP 324,274.

The coding sequence for hu-MIP-2β was obtained by polymerase chain reaction (PCR) amplification of a fragment derived from a λgt10 clone (λgt10-hu-MIP-2-4a) using the following primers: 
5' Primer:
5'-GAGTGCGGTACCCTTGGATAAAAGAGACGTCCGTGGTCACTGAACTGCGCTGCC - 3'
↑ |→hu-MIP-2β
KpnI

3'Primer:
5'-GAGTGCGTCGACTCATCAGTTGGTGCTCCCCTTGTTCAG - 3'
↑ | ←hu-MIP-2β
SAlI stop
After 30 cycles of PCR amplification, the DNA was digested with KpnI and SalI, and the 244 bp fragment encoding the 4 carboxy-terminal amino acids of the α-factor leader, the dibasic processing site, and the entire 73 amino acid mature hu-MIP-2β was isolated by acrylamide gel electrophoresis. This fragment was then ligated into pGAI1 that had been digested with KpnI and SalI and purified on an agarose gel. Following bacterial transformation and screening, plasmid pMIP540 was obtained which upon DNA sequencing was found to have the predicted nucleotide sequence. This plasmid was digested with BamHI and the resulting 1163 pb fragment including the GAPDH promoter sequence, the α-factor leader/hu-MIP-2β fusion protein, and the α-factor transcriptional terminator was cloned into the BamHI site of expression vector pAB24 to provide expression plasmid pYMIP540.

Saccharomyces cerevisiae strain MB2-1 (leu2-3, leu2-112, his3-11, his3-15, ura3Δ, CAN, Cir°) was transformed with plasmid pYMIP540 by standard procedures and transformants selected for ura prototrophy. Expression was analyzed by inoculation of single colonies of individual transformants into leucine selective medium and growing for about 48 hours. Cultures were then centrifuged, cells resuspended in medium lacking uracil, and diluted 20x into ura selective medium. Cultures were then grown for about 72 hours, then harvested and cell-free supernatants prepared. Conditioned medium was analyzed for the presence of hu-MIP-2β by SDS-PAGE followed by Coomassie staining. A band was observed on SDS-PAGE which migrated similar to a native mMIP-2 standard (provided by B Sherry, Rockefeller University).

### Example 6

### Expression in Mammalian Cells

### (A) Expression in COS-7 Cells

Plasmid pSV7tPA21 (ATCC Accession No. 40163, deposited 14 February 1985) is a mammalian expression vector containing DNA encoding full-length human tPA in a polylinker sequence flanked by the SV40 origin, early promoter, and polyadenylation site. This plasmid is also described in PCT Application WO 86/05514.

The sequence encoding hu-MIP-2β is amplified from plasmid pYMIP540 by PCR and inserted into a site of expression vector pSV7tPA2I having an SV40 origin and early promoter 5' of the site and a polyadenylation site 3' of the insertion site to form plasmid pSV-MIP2β. COS-7 cells are transfected with pSV-MIP2β using a modification of the procedure described by Graham and van der Eb., Virol. (1973), 52:456-67. The samples are added to the dishes in duplicate and allowed to settle onto the cells for 6 hours in a CO₂ incubator at 37°C. After culturing, the cells are rinsed gently with calcium- and magnesium-free PBS. The dishes are then exposed to glycerol as an adjuvant for 3-4 minutes, rinsed, and fed with DMEM medium supplemented with 4.5 mg/mL glucose, 3.7 mg/mL sodium bicarbonate, 292 »g/mL glutamine, 110 »g/mL sodium pyruvate, 100 U/mL penicillin, 100 U/mL streptomycin, and 10% fetal calf serum (FCS). After allowing the culture to recover from the glycerol shock, the medium is replaced with serum-free medium. Twelve hours after serum withdrawal, the conditioned medium is assayed for hu-MIP-2β.

### (B) Expression in CHO Cells

CHO dhfr⁻ cells are plated at a density of 5x10⁵ to 10⁶ cells/dish (10 cm) the day prior to transfection in F12 medium supplemented with 1.18 mg/mL NaHCO₃, 292 »g/mL glutamine, 110 »g/mL sodium pyruvate, 100 U/mL penicillin, 100 U/mL streptomycin, 150 mg/mL proline, and 10% FCS. The cells are then transfected as described in part (A) above, further including a marker plasmid bearing a dhfr gene linked to the adenovirus major late promoter (coprecipitated in calcium phosphate). After culture for 48 hours, the cells are split 1:20, grown in selective medium (DMEM, 150 »g/mL proline, and 10% FCS), and cultured for 1-2 weeks.

### Example 7

### A. Preparation of a plasmid encoding a selectable marker

The plasmid pAd-DHFR, bearing the murine DHFR cDNA, was constructed by fusing the major late promoter from adenovirus-2 (Ad-MLP, map units 16-27.3) to the 5' untranslated sequences of the mouse DHFR cDNA (J.H. Nunberg et al, Cell (1980) 19:355-64). SV40 DNA encoding part of the early transcription unit, including the intron of the small t antigen gene, and having the SV40 early region transcriptional termination region, was obtained from pSV2-neo (Southern and Berg, J. Mol. Appl. Gen. (1982) 1:327-41) and fused to the 3' untranslated end of the DHFR cDNA. These three segments were subcloned into pBR322 to obtain plasmid pAD-DHFR.

### B. Preparation of Mammalian Expression Vector

### 1. pSV7d:

The expression cassettes were prepared using the mammalian cell expression vector pSV7d (2423 bp).

The plasmid pSV7d (see Truett et al, 1985, DNA, 4:333) was constructed as follows: The 400 bp BamHI/HindIII fragment containing the SV90 origin of replication and early promoter was excised from pSVgtI (obtained from Paul Berg, Stanford University, California) and purified. The 240 bp SV40 Bc1I/BamHI fragment containing the SV40 polyA addition site was excised from psV2/DHFR (Subramani et al, Mol. Cell. Biol. (1981) 1:854-864) and purified. The fragments were fused through the following linker: 
Stop Codons

1 2 3

5'-AGCTAGATCTCCCGGGTCTAGATAAGTAAT-3'
| TCTAGAGGGCCCAGATCTATTCATTACTAG
| | | |
HindIII BglII SmaI XbaI BclI overhang.
This linker contains five restriction sites, as well as stop codons in all three reading frames. The resulting 670 bp fragment containing the SV40 origin of replication, the SV40 early promoter, the polylinker with stop codons and the SV40 polyadenylation site was cloned into the BamHI site of pML, a pBR322 derivative having about 1.5 Kb deleted (Lusky and Botchan, Cell (1984) 36:391), to yield pSV6. The EcoRI and EcoRV sites in the pML sequences of psV6 were eliminated by digestion with EcoRI and EcoRV, treated with Ba131 nuclease to remove about 200 bp on each end, and finally religated to yield PSV7a. The Ba131 resection also eliminated one BamHI restriction site flanking the SV40 region, approximately 200 bp away from the EcoRV site. To eliminate the second BamHI site flanking the SV40 region, pSV7a was digested with NruI, which cuts in the pML sequence upstream from the origin of replication. This was recircularized by blunt end ligation to yield pSV7b.

pSV7c and pSV7d represent successive polylinker replacements. First, pSV7b was digested with StuI and XbaI. Then, the following linker was ligated into the vector to yield pSV7c: 
BglII  EcoRI  SmaI  KpnI  XbaI
5'-AGATCTCGAATTCCCCGGGGTACCT
   TCTAGAGCTTAAGGGGCCCCATGGAGATC
Thereafter, pSV7c was digested with Bg1II and XbaI, and then ligated with the following linker to yield psV7d:

BglII    EcoRI SmaI    XbaI   BamHI SalI
|        |     |       |      |     |
5'-GATCTCGAATTCCCCGGGTCTAGAGGATCCGTCGAC
       AGCTTAAGGGGCCCAGATCTCCTAGGCACGTGGATC

### 2. pCMV6a

In an effort to improve the level of transcription and stability of the messenger RNA of hetereologous proteins, the SV40 early transcriptional initiation region was replaced by sequences from the human cytomegalovirus immediate early region (Boshart et al, Cell (1985) 4:521-530). In addition, 5' untranslated sequences contributed by the SV40 early region to the messenger RNA were replaced with the 5' untranslated sequences of the HCMV 1E1 gene, including its first intron. This intron is included on the assumption that spliced transcripts lead to faster processing and more stable mRNA. The expression vector also has an SV40 origin of replication to permit transient expression in COS7 cells, and a bacterial β-lactamase gene to permit DNA cloning by selection for ampicillin resistance.

The plasmid was constructed from a 700 bp SalI-PvuI fragment of pSV7d (described in Example 7, Section B, 1) containing the SV40 polyadenylation region, a 1400 bp PvuI-EcoRI (filled in with Klenow polymerase) fragment of pSVT2 (Myers et al., Cell (1981) 25:373-84; Rio et al., Cell (1983) 32:1227-40) providing the SV40 origin of replication and the rest of the β-lactamase gene, a 1700 bp SspI-SalI fragment derived from a plasmid subclone of the human cytomegalovirus (Towne strain) in which the SalI site was introduced by in vitro mutagenesis near the translational start site for the 1E1 protein, and the 4300 bp SalI-SalI fragment of pSVF8-92C containing the cDNA encoding the Factor VIII:C 92 K Mᵣ glycoprotein.

pCMV6a120-SF2 plasmid is similar to pCMV6a described above except that the SalI-SalI fragment encoding the Factor VIII:C 92K Mᵣ protein was replaced with an expression cassette containing the 5' untranslated and signal sequence of human tPA fused to a heterologous gene. This vector was transformed into E.coli cells that are deposited with the ATCC, accession number 68249. Digestion of pCMV6a120-SF2 with NheI and SalI will remove the heterologous gene. Any hu-MIP-2β polypeptide gene can be ligated to linkers to be inserted into the mammalian expression vector pCMV6a120-SF2. For example, the hu-MIP-2β gene is obtained from the yeast plasmid by polymerase chain reaction (PCR) amplification. Primers to direct the PCR reaction can easily be constructed based on the nucleic acid sequence of hu-MIP-2β shown in Figure 3, and the primers can be constructed to include appropriate restriction sites, such as NheI and SalI, for inserting the amplified gene into the vector. For convenience, the DHFR gene can be inserted into this new hu-MIP-2β expression cassette. This new vector is called pCMV-MIP2β.

### C. Preparation of a MIP-2β Mammalian Expressing Cell Line

This example describes the preparation of a stable CHO cell line that produces the native hu-MIP-2β.

The DHFR⁻ CHO cell line DG44 (G. Urlaub et al., Som. Cell. Mol. Genet. (1986) 12:555-66) is first transfected with the plasmid pCMV-MIP-2β. In this plasmid the CMV promoter (described in Example 7B-2) regulates MIP-2β gene derived from pYMIP550 (described in Example 5), the tPA leader (described in Example 7B-2) directs the secretion of the encoded hu-MIP-2β, and contains downstream the Ad-MLP/dhfr cassette derived from pAd-DHFR (described in Example 7A). The cells are transfected using the polybrene method described by W. Chaney et al., Som. Cell. Mol. Genet (1986) 12:237-44. By selecting for DHFR⁺ cells (in DMEM + 10% DFSC) several hu-MIP-2β producers are isolated.

### Deposit Information

The following materials were deposited with the American Type Culture Collection (ATCC), 12301 Parklawn Drive, Rockville, Maryland 20852:

| Name | Deposit Date | Acces. No. |
|---|---|---|
| Chinese Hamster Ovary Cells CHO-DG44 | 8 March 1990 | CRL 10378 |
| E. coli HB101 pCMV6a120-SF2 | 8 March 1990 | 68249 |
| S. cerevisiae MB2-1 (pYMIP540) | 20 June 1990 | 74002 |

These materials were deposited under the terms of the Budapest Treaty on the International Recognition of the Deposit of Micro-organisms for Purposes of Patent Procedure. These deposits are provided as a convenience to those of skill in the art, and do not represent an admission that a deposit is required under 35 U.S.C. Section 112. The sequence of the polynucleotides contained in the deposited materials, as well as the amino acid sequence of the polypeptides encoded thereby, are incorporated herein by reference and are controlling in the event of any conflict with the written description of sequences herein. A license may be required to make, use or sell the deposited materials, and no such license is hereby granted.

## Claims

1. A composition comprising human MIP-2β polypeptide substantially free of human tissue, said polypeptide selected from the group consisting of human MIP-2β, human MIP-2β muteins, truncated human MIP-2β peptides and human MIP-2β fusion proteins, said polypeptide containing a sequence of amino acids which is found in Figure 3 but not found in human MGSA, human MIP-2α, murine MIP-2, or murine KC.

2. The composition of claim 1 which is substantially free of other human proteins.

3. The composition of claim 1 which is substantially free of other protein.

4. The composition of claim 1 wherein said hu-MIP-2β polypeptide is hu-MIP-2β.

5. A composition comprising DNA molecules comprising a heterologous region that encodes hu-MIP-2β polypeptide said polypeptide selected from the group consisting of human MIP-2β, human MIP-2β muteins, truncated human MIP-2β peptides and human MIP-2β fusion proteins, said polypeptide containing a sequence of amino acids which is found in Figure 3 but not found in human MGSA, human MIP-2α, murine MIP-2, or murine KC, wherein the composition is substantially free of other DNA molecules.

6. The composition of claim 5 wherein said region encoding hu-MIP-2β polypeptide is an intron-free DNA sequence.

7. A DNA molecule comprising an intron-free DNA sequence encoding an amino acid sequence of hu-MIP-2β polypeptide said polypeptide selected from the group consisting of human MIP-2β, human MIP-2β muteins, truncated human MIP-2β peptides and human MIP-2β fusion proteins, said polypeptide containing a sequence of amino acids which is found in Figure 3 but not found in human MGSA, human MIP-2α, murine MIP-2, or murine KC.

8. A DNA molecule according to claim 7 wherein said hu-MIP-2β polypeptide is hu-MIP-2β.

9. A cell population transformed with the DNA molecule of claim 5, said population being substantially free of cells not transformed with said DNA molecule.

10. The method of producing a hu-MIP-2β polypeptide, said polypeptide selected from the group consisting of human MIP-2β, human MIP-2β muteins, truncated human MIP-2β peptides and human MIP-2β fusion proteins, said polypeptide containing a sequence of amino acids which is found in Figure 3 but not found in human MGSA, human MIP-2α, murine MIP-2, or murine KC, comprising:
providing a population of transformed cells of claim 9;
growing said population under conditions whereby said polypeptide is expressed; and
recovering said polypeptide.

11. The method of claim 10 where said polypeptide is excreted by said cell.

12. A single stranded DNA molecule comprising at least 20 sequential nucleotides, wherein said sequential nucleotides comprise a subsequence unique to the sequence of hu-MIP-2β shown in Figure 3 or a DNA sequence complementary thereto.

13. A method for determining the presence of a polynucleotide substantially homologous to a coding sequence for human MIP-2β comprising:
providing a sample suspected of comprising said polynucleotide;
incubating the sample with a nucleotide probe having a sequence complementary to the single stranded DNA of claim 12, under conditions where said probe will form hybrids with nucleic acid from the sample; and
detecting nucleic acid hybrids.

14. An antibody reactive with an epitope on a hu-MIP-2β polypeptide said polypeptide selected from the group consisting of human MIP-2β, human MIP-2β muteins, truncated human MIP-2β peptides and human MIP-2β fusion proteins, said polypeptide containing a sequence of amino acids which is found in Figure 3 but not found in human MGSA, human MIP-2α, murine MIP-2, or murine KC but not reactive with murine MIP-2, human gro protein or the protein encoded by the murine KC gene.

15. The antibody of claim 14 wherein said antibody is monoclonal.

16. A composition comprising the antibody of claim 14 wherein said composition is substantially free of other immunoglobulin molecules.

17. A hybridoma cell line which produces the monoclonal antibody of claim 15.

18. A method for determining the presence of human MIP-2β in a sample comprising:
incubating said sample with an antibody reactive with hu-MIP-2β polypeptide said polypeptide selected from the group consisting of human MIP-2β, human MIP-2β muteins, truncated human MIP-2β peptides and human MIP-2β fusion proteins, said polypeptide containing a sequence of amino acids which is found in Figure 3 but not found in human MGSA, human MIP-2α, murine MIP-2, or murine KC; and
detecting immunocomplex.

19. A method for determining the presence of anti-hu-MIP-2β antibodies in a sample, comprising:
incubating said sample with the composition of claim 2; and
detecting immunocomplex.

20. A pharmaceutical composition stimulating myelopoiesis in myelopoietic cells comprising an effective amount of hu-MIP-2β polypeptide said polypeptide selected from the group consisting of human MIP-2β, human MIP-2β muteins, truncated human MIP-2β peptides and human MIP-2β fusion proteins, said polypeptide containing a sequence of amino acids which is found in Figure 3 but not found in human MGSA, human MIP-2α, murine MIP-2, or murine KC.

## Patentansprüche

1. Mittel, das menschliches MIP-2β-Polypeptid enthält und im wesentlichen frei von menschlichem Gewebe ist, wobei das Polypeptid aus menschlichem MIP-2β, menschlichen MIP-2β-Muteinen, verkürzten menschlichen MIP-2β-Peptiden und menschlichen MIP-2β-Fusionsproteinen ausgewählt ist und eine Aminosäuresequenz, die in Figur 3 dargestellt ist, enthält, die aber nicht in menschlichem MGSA, menschlichem MIP-2α, Maus-MIP-2 oder Maus-KC vorkommt.

2. Mittel nach Anspruch 1, das im wesentlichen frei von anderen menschlichen Proteinen ist.

3. Mittel nach Anspruch 1, das im wesentlichen frei von anderen Proteinen ist.

4. Mittel nach Anspruch 1, wobei das hu-MIP-2β-Polypeptid hu-MIP-2β ist.

5. Mittel, enthaltend DNA-Moleküle, die einen heterologen Bereich umfassen, der hu-MIP-2β-Polypeptid codiert, wobei das Polypeptid aus menschlichem MIP-2β, menschlichen MIP-2β-Muteinen, verkürzten menschlichen MIP-2β-Peptiden und menschlichen MIP-2β-Fusionsproteinen ausgewählt ist und eine Aminosäuresequenz, die in Figur 3 dargestellt ist, enthält, die aber nicht in menschlichem MGSA, menschlichem MIP-2α, Maus-MIP-2 oder Maus-KC vorkommt, wobei das Mittel im wesentlichen frei von anderen DNA-Molekülen ist.

6. Mittel nach Anspruch 5, wobei der das hu-MIP-2β-Polypeptid codierende Bereich eine intronfreie DNA-Sequenz ist.

7. DNA-Molekül, das eine intronfreie DNA-Sequenz enthält, die eine Aminosäuresequenz des hu-MIP-2β-Polypeptids codiert, wobei das Polypeptid aus menschlichem MIP-2β, menschlichen MIP-2β-Muteinen, verkürzten menschlichen MIP-2β-Peptiden und menschlichen MIP-2β-Fusionsproteinen ausgewählt ist und eine Aminosäuresequenz, die in Figur 3 dargestellt ist, enthält, die aber nicht in menschlichem MGSA, menschlichem MIP-2α, Maus-MIP-2 oder Maus-KC vorkommt.

8. DNA-Molekül nach Anspruch 7, wobei das hu-MIP-2β-Polypeptid hu-MIP-2β ist.

9. Zellpopulation, die mit dem DNA-Molekül nach Anspruch 5 transformiert ist, wobei die Population im wesentlichen frei von Zellen ist, die nicht mit dem DNA-Molekül transformiert wurden.

10. Verfahren zur Herstellung eines hu-MIP-2β-Polypeptids, wobei das Polypeptid aus menschlichem MIP-2β, menschlichen MIP-2β-Muteinen, verkürzten menschlichen MIP-2β-Peptiden und menschlichen MIP-2β-Fusionsproteinen ausgewählt ist und eine Aminosäuresequenz, die in Figur 3 dargestellt ist, enthält, die aber nicht in menschlichem MGSA, menschlichem MIP-2α, Maus-MIP-2 oder Maus-KC vorkommt, umfassend:
Bereitstellung einer Population von transformierten Zellen nach Anspruch 9,
Züchtung der Population unter Bedingungen, unter denen das Polypeptid exprimiert wird, und
Gewinnung des Polypeptids.

11. Verfahren nach Anspruch 10, wobei das Polypeptid von der Zelle sezerniert wird.

12. Einzelstrang-DNA-Molekül, umfassend mindestens 20 aufeinanderfolgende Nucleotide, wobei die aufeinanderfolgenden Nucleotide eine Untersequenz umfassen, die einzigartig ist für die Sequenz von hu-MIP-2β, wie sie in Figur 3 gezeigt ist, oder eine dazu komplementäre DNA-Sequenz.

13. Verfahren zum Nachweis der Gegenwart eines Polynucleotids, das im wesentlichen homolog zur Codierungssequenz für menschliches MIP-2β ist, umfassend:
Bereitstellung einer Probe, von der man annimmt, daß sie das Polynucleotid enthält,
Inkubation der Probe mit einer Nucleotidsonde, die eine Sequenz aufweist, die komplementär zur Einzelstrang-DNA nach Anspruch 12 ist, unter Bedingungen, bei denen die Sonde Hybride mit Nucleinsäure aus der Probe bildet, und Nachweis der Nucleinsäurehybride.

14. Antikörper, der mit einem Epitop auf einem hu-MIP-2β-Polypeptid reagiert, wobei das Polypeptid aus menschlichem MIP-2β, menschlichen MIP-2β-Muteinen, verkürzten menschlichen MIP-2β-Peptiden und menschlichen MIP-2β-Fusionsproteinen ausgewählt ist und eine in Figur 3 dargestellte Aminosäuresequenz enthält, die aber nicht in menschlichem MGSA, menschlichem MIP-2α, Maus-MIP-2 oder Maus-KC vorkommt, aber nicht mit Maus-MIP-2, menschlichem gro-Protein oder dem durch das Maus-KC-Gen codierten Protein reagiert.

15. Antikörper nach Anspruch 14, wobei der Antikörper ein monoclonaler Antikörper ist.

16. Mittel, das den Antikörper nach Anspruch 14 enthält, wobei das Mittel im wesentlichen frei von anderen Immunglobulinmolekülen ist.

17. Hybridomzellinie, die den monoclonalen Antikörper nach Anspruch 15 produziert.

18. Verfahren zum Nachweis der Gegenwart von menschlichem MIP-2β in einer Probe, umfassend:
Inkubation der Probe mit einem Antikörper, der mit hu-MIP-2β-Polypeptid reagiert, wobei das Polypeptid aus menschlichem MIP-2β, menschlichen MIP-2β-Muteinen, verkürzten menschlichen MIP-2β-Peptiden und menschlichen MIP-2β-Fusionsproteinen ausgewählt ist und eine Aminosäuresequenz, die in Figur 3 dargestellt ist, enthält, die aber nicht in menschlichem MGSA, menschlichem MIP-2α, Maus-MIP-2 oder Maus-KC vorkommt, und
Nachweis des Immunkomplexes.

19. Verfahren zum Nachweis der Gegenwart von anti-hu-MIP-2β-Antikörpern in einer Probe, umfassend:
Inkubation der Probe mit dem Mittel nach Anspruch 2 und Nachweis des Immunkomplexes.

20. Arzneimittel, das die Knochenmarkbildung in myelopoetischen Zellen stimuliert, umfassend eine wirksame Menge des hu-MIP-2β-Polypeptids, wobei das Polypeptid aus menschlichem MIP-2β, menschlichen MIP-2β-Muteinen, verkürzten menschlichen MIP-2β-Peptiden und menschlichen MIP-2β-Fusionsproteinen ausgewählt ist und eine Aminosäuresequenz, die in Figur 3 dargestellt ist, enthält, die aber nicht in menschlichem MGSA, menschlichem MIP-2α, Maus-MIP-2 oder Maus-KC vorkommt.

## Revendications

1. Composition comprenant le polypeptide MIP-2β humain pratiquement exempt de tissu humain, ledit polypeptide étant choisi dans le groupe comprenant MIP-2β humain, les mutéines de MIP-2β humain, les peptides tronqués de MIP-2β humain, et les protéines de fusion de MIP-2β humain, ledit polypeptide contenant une séquence d'acides aminés que l'on trouve sur la figure 3 mais que l'on ne trouve pas dans MGSA humain, MIP-2α humain, MIP-2 murin ou KC murin.

2. Composition selon la revendication 1, qui est pratiquement exempte d'autres protéines humaines.

3. Composition selon la revendication 1, qui est pratiquement exempte d'autres protéines.

4. Composition selon la revendication 1, dans laquelle ledit polypeptide hu-MIP-2β est hu-MIP-2β.

5. Composition comprenant des molécules d'ADN comprenant une région hétérologue qui code pour le polypeptide hu-MIP-2β, ledit polypeptide étant choisi dans le groupe comprenant MIP-2β humain, les mutéines de MIP-2β humain, les peptides tronqués de MIP-2β humain et les protéines de fusion de MIP-2β humain, ledit polypeptide contenant une séquence d'acides aminés que l'on trouve sur la figure 3 mais que l'on ne trouve pas dans MGSA humain, MIP-2α humain, MIP-2 murin ou KC murin, laquelle composition est pratiquement exempte d'autres molécules d'ADN.

6. Composition selon la revendication 5, dans laquelle ladite région codant pour le polypeptide hu-MIP-2β est une séquence d'ADN exempte d'intron.

7. Molécule d'ADN comprenant une séquence d'ADN exempte d'intron codant pour une séquence d'acides aminés du polypeptide hu-MIP-2β, ledit polypeptide étant choisi dans le groupe comprenant MIP-2β humain, les mutéines de MIP-2β humain, les peptides tronqués de MIP-2β humain et les protéines de tusion de MIP-2β humain, ledit polypeptide contenant une séquence d'acides aminés que l'on trouve sur la figure 3 mais que l'on ne trouve pas dans MGSA humain, MIP-2α humain, MIP-2 murin ou KC murin.

8. Molécule d'ADN selon la revendication 7, dans laquelle ledit polypeptide hu-MIP-2β est hu-MIP-2β.

9. Population cellulaire transformée avec la molécule d'ADN de la revendication 5, ladite population étant pratiquement exempte de cellules non transformées avec ladite molécule d'ADN.

10. Procédé de production d'un polypeptide hu-MIP-2β, ledit polypeptide étant choisi dans le groupe comprenant MIP-2β humain, les mutéines de hu-MIP-2β humain, les peptides tronqués de MIP-2β humain et les protéines de fusion de MIP-2β humain, ledit polypeptide contenant une séquence d'acides aminés que l'on trouve sur la figure 3 mais que l'on ne trouve pas dans MGSA humain, MIP-2α humain, MIP-2 murin ou KC murin, qui comprend :
- la fourniture d'une population de cellules transformées selon la revendication 9 ;
- la croissance de ladite population dans des conditions dans lesquelles ledit polypeptide est exprimé ; et
- la récupération dudit polypeptide.

11. Procédé selon la revendication 10, dans lequel ledit polypeptide est excrété par lesdites cellules.

12. Molécule d'ADN monocaténaire comprenant au moins 20 nucléotides séquentiels, dans laquelle lesdits nucléotides séquentiels comprennent une sous-séquence unique à la séquence de hu-MIP-2β, représenté sur la figure 3 ou une séquence d'ADN complémentaire à cela.

13. Procédé pour déterminer la présence d'un polynucléotide pratiquement homologue à une séquence codante pour hu-MIP-2β, comprenant :
- la fourniture d'un échantillon suspecté de comprendre ledit polynucléotide ;
- l'incubation de l'échantillon avec une sonde nucléotidique ayant une séquence complémentaire à l'ADN monocaténaire de la revendication 12, dans des conditions dans lesquelles ladite sonde formera des hybrides avec l'acide nucléique de l'échantillon ; et
- la détection des hybrides de l'acide nucléique.

14. Anticorps réactif avec un épitope sur un polypeptide hu-MIP-2β, ledit polypeptide étant choisi dans le groupe comprenant MIP-2β humain, les mutéines de MIP-2β humain, les peptides tronqués de MIP-2β humain, et les protéines de fusion de MIP-2β humain, ledit polypeptide contenant un séquence d'acides aminés que l'on trouve sur la figure 3 mais que l'on ne trouve pas dans MGSA humain, MIP-2α humain, MIP-2 murin ou KC murin, mais non réactif avec MIP-2 murin, la protéine gro humaine ou la protéine codée par le gène KC murin.

15. Anticorps selon la revendication 14, dans lequel ledit anticorps est monoclonal.

16. Composition comprenant l'anticorps selon la revendication 14 dans laquelle ladite composition est pratiquement exempte d'autres molécules d'immunoglobuline.

17. Lignée cellulaire hybridome qui produit l'anticorps monoclonal selon la revendication 15.

18. Procédé pour déterminer la présence de MIP-2β humain dans un échantillon, qui comprend :
l'incubation dudit échantillon avec un anticorps réactif avec le polypeptide hu-MIP-2β, ledit polypeptide étant choisi dans le groupe comprenant MIP-2β humain, les mutéines de MIP-2β humain, les peptides tronqués MIP-2β humain et les protéines de fusion de MIP-2β humain, ledit polypeptide contenant une séquence d'acides aminés que l'on trouve sur la figure 3 mais que l'on ne trouve pas dans MGSA humain, MIP-2α humain, MIP-2 murin ou KC murin ; et
la détection du complexe immun.

19. Procédé pour déterminer la présence d'anticorps anti-hu-MIP-2β dans un échantillon, qui comprend :
l'incubation dudit échantillon avec la composition de la revendication 2 ; et
la détection du complexe immun.

20. Composition pharmaceutique stimulant la myélopoïèse dans des cellules myélopoïétiques, comprenant une quantité efficace de polypeptide hu-MIP-2β, ledit polypeptide étant choisi dans le groupe comprenant MIP-2β humain, les mutéines de MIP-2β humain, les peptides tronqués de MIP-2β humain et les protéines de fusion de MIP-2β humain, ledit polypeptide contenant une séquence d'acides aminés que l'on trouve sur la figure 3 mais que l'on ne trouve pas dans MGSA humain, MIP-2α humain, MIP-2 murin ou KC murin.
